# EUROPEAN PATENT SPECIFICATION

(11) **EP 3 675 773 B1**
(45) Date of publication and mention of the grant of the patent: **09.12.2026**
(21) Application number: 18752939.1
(22) Date of filing: 24.07.2018
(51) Int. Cl.: A61F 2/24

(54) **PROSTHETIC LEAFLET DEVICE**
KLAPPENPROTHESENVORRICHTUNG
DISPOSITIF DE FEUILLET PROTHÉTIQUE

(30) Priority: 31.08.2017 US 201762552595 P; 07.11.2017 US 201762582519 P
(43) Date of publication of application: 08.07.2020
(73) Proprietor: Medtronic, Inc., Minneapolis, MN 55432 (US)
(72) Inventor: MCLEAN, Matt, Menlo Park, CA 94025 (US); GIFFORD, Hanson S., III, Menlo Park, CA 94025 (US); FANN, James I., Menlo Park, CA 94025 (US); SUTTON, Douglas, Menlo Park, CA 94025 (US)
(74) Representative: Zimmermann & Partner Patentanwälte mbB
(86) International application number: PCT/US2018/043566
(87) International publication number: WO 2019/045910

(56) References cited:
- EP-A1- 3 167 846
- WO-A2-2006/032051
- DE-A1- 102013 017 750
- US-A1- 2003 199 975
- US-A1- 2010 262 233

## Description

### Cross-Reference to Related Applications

The present application claims the benefit of and priority to U.S. Provisional Patent Application No. 62/552,595, file August 31, 2017, and U.S. Provisional Patent Application No. 62/582,519, filed November 7, 2017.

### Field of the Invention

The present technology relates generally to implants for repairing a regurgitant or incompetent cardiac valve and for methods of implanting the same. The present technology is particularly useful for repairing a regurgitant mitral valve.

### Background

Conditions affecting the proper functioning of the mitral valve include, for example, mitral valve regurgitation, mitral valve prolapse and mitral valve stenosis. Mitral valve regurgitation is a disorder of the heart in which the leaflets of the mitral valve fail to coapt into apposition at peak contraction pressures, resulting in abnormal leaking of blood from the left ventricle into the left atrium. There are a number of structural factors that may affect the proper closure of the mitral valve leaflets. For example, many patients suffering from heart disease experience dilation of the heart muscle, resulting in an enlarged mitral annulus. Enlargement of the mitral annulus makes it difficult for the leaflets to coapt during systole. A stretch or tear in the chordae tendineae, the tendons connecting the papillary muscles to the inferior side of the mitral valve leaflets, may also affect proper closure of the mitral annulus. A ruptured chordae tendineae, for example, may cause a valve leaflet to prolapse into the left atrium due to inadequate tension on the leaflet. Abnormal backflow can also occur when the functioning of the papillary muscles is compromised, for example, due to ischemia. As the left ventricle contracts during systole, the affected papillary muscles do not contract sufficiently to effect proper closure.

Mitral valve prolapse, or when the mitral leaflets bulge abnormally up in to the left atrium, causes irregular behavior of the mitral valve and may also lead to mitral valve regurgitation. Normal functioning of the mitral valve may also be affected by mitral valve stenosis, or a narrowing of the mitral valve orifice, which causes impedance of filling of the left ventricle in diastole.

Typically, treatment for mitral valve regurgitation has involved the application of diuretics and/or vasodilators to reduce the amount of blood flowing back into the left atrium. Other procedures have involved surgical approaches (open and intravascular) for either the repair or replacement of the valve. For example, typical repair approaches have involved cinching or resecting portions of the dilated annulus.

Cinching of the annulus has been accomplished by the implantation of annular or peri-annular rings which are generally secured to the annulus or surrounding tissue. Other repair procedures have also involved suturing or clipping of the valve leaflets into partial apposition with one another.

Alternatively, more invasive procedures have involved the replacement of the entire valve itself where mechanical valves or biological tissue are implanted into the heart in place of the mitral valve. These invasive procedures are conventionally done through large open thoracotomies and are thus very painful, have significant morbidity, and require long recovery periods.

However, with many repair and replacement procedures, the durability of the devices or improper sizing of annuloplasty rings or replacement valves may result in additional problems for the patient. Moreover, many of the repair procedures are highly dependent upon the skill of the cardiac surgeon where poorly or inaccurately placed sutures may affect the success of procedures.

In addition to its irregular, unpredictable shape, the mitral valve annulus lacks a significant amount of radial support from surrounding tissue. The mitral valve is bound by muscular tissue on the outer wall only. The inner wall of the mitral valve is bound by a thin vessel wall separating the mitral valve annulus from the inferior portion of the aortic outflow tract. As a result, significant radial forces on the mitral annulus could lead to collapse of the inferior portion of the aortic tract with potentially fatal consequences.

The chordae tendineae of the left ventricle may also present an obstacle in deploying a mitral valve repair device. The maze of chordae in the left ventricle makes navigating and positioning a deployment catheter that much more difficult in mitral valve repair. US 2003/199975 A1 discloses an apparatus for assisting operation of a heart valve includes a generally arcuate base and a buttress extending generally from at least a portion of the base. The apparatus can be deformable to facilitate its implantation. When the apparatus is implanted at an annulus of a heart valve, the buttress thereof cooperates with one or more leaflets of the heart valve to help control blood flow relative to the apparatus and heart valve. WO 2006/032051 A2 relates to a device for treatment of heart valve regurgitation, the device can be implanted within a heart to at least partially block gaps that may be present between to mitral leaflets.

Given the difficulties associated with current procedures, there remains the need for simple, effective, and less invasive devices and methods for treating dysfunctional heart valves. The claimed subject matter is defined in the preceding claims.

Disclosed is a heart valve repair device, comprising an atrial-fixation member having an expandable mesh configured to have oval or circular shape in a deployed configuration, the atrial-fixation member defining a central lumen; and a baffle extending from a portion of the atrial-fixation member, the baffle having an anterior portion with a smooth, atraumatic surface configured to coapt with at least a portion of one or more native leaflets of a native heart valve, a posterior portion configured to engage and immobilize at least a portion of another native leaflet of the native heart valve, wherein the baffle extends radially inward from the atrial-fixation member into the central lumen to approximate a closed position of the immobilized leaflet.

In the aforementioned device, the baffle having struts configured into a basket having a hollow interior volume, the baffle extending from a portion of the atrial-fixation member, wherein the baffle includes a smooth, atraumatic coaptation surface facing the central lumen which defines a prosthetic coaptation surface for at least a portion of one or more native leaflets, and a restraining portion configured to engage and restrain at least a first portion of a functionally deficient native leaflet while leaving a second portion of the functionally deficient native leaflet mobile, the baffle extends radially inward from the into the central lumen to approximate a closed position for the functionally deficient leaflet.

A device according to any of the preceding further comprising a plurality of frictional elements provided on portions of the atrial-fixation member or the baffle.

The preceding device, wherein the medial and lateral sides of the atrial-fixation member do not include any frictional elements.

A device according to any of the preceding, wherein the baffle comprises plurality of anterior struts and a plurality of posterior struts with gaps interposed between adjacent ones of the anterior and posterior struts.

A device according to any of the preceding, comprising: a fabric covering at least partially surrounding the baffle; and the basket has a mouth which is not covered by the fabric.

A device according to any of the preceding, comprising: a fabric covering at least partially surrounding the baffle; the basket has a mouth which is covered by the fabric.

A device according to any of the preceding, wherein the baffle comprises a biocompatible foam.

A device according to any of the preceding, wherein the anterior portion of the baffle has a convex shape.

A device according to any of the preceding, wherein the posterior portion of the baffle is sized and configured to engage with a central scallop of an anterior or a posterior mitral valve leaflet having three scallops, while leaving the remaining two scallops mobile.

A device according to any of the preceding, further comprising: a first set of plural suture loops circumferentially disposed around the atrial-fixation member, each suture loop of the first set of suture loops having a lumen; and a first suture disposed in the lumen of the suture loops and interconnecting adjacent suture loops; wherein a diameter of the atrial-fixation member is adjusted by cinching the first suture.

A device according to any of the preceding, further comprising: a second set of plural suture loops circumferentially disposed around the baffle, each suture loop of the second set of suture loops having a lumen; and a second suture disposed in the lumen of the suture loops and interconnecting adjacent suture loops; wherein a diameter of the baffle is adjusted by cinching the second suture.

A device according to any of the preceding, further comprising: a third suture loop provided on the baffle, a third suture disposed in the lumen of the third suture loop; wherein the pulling on the third suture displaces the baffle.

A device according to any of the preceding, further comprising at least one fabric segment of fabric attached to and at least partially spanning the atrial-fixation member, wherein the fabric covering facilitates tissue ingrowth.

A device according to any of the preceding, further comprising at least one fabric segment of fabric depending from the distal end of the atrial-fixation member. The device may further comprise a biasing member attached to the at least one fabric segment and biasing the fabric segment away from the central lumen.

The device according to any of the preceding, further comprising a segment of fabric attached to the atrial-fixation member, wherein the fabric covering facilitates tissue ingrowth.

The device according to any of the preceding, wherein the atrial-fixation member comprises at least two rows of cells.

The device according to any of the preceding, wherein: the atrial-fixation member further comprises a row of chevrons; at least two of the chevrons include a through-hole; and a suture threaded through the through-hole; wherein cinching the suture adjusts a diameter of the atrial-fixation member.

The device according to any of the preceding, wherein the atrial-fixation member has a frustoconical shape. The atrial-fixation member may be configured to engage solely with a wall of the atrium. The size of the atrial-fixation member unconstrained by external forces is larger than a size of the atrium in diastole. The device may be fixed relative to the native cardiac valve solely by the atrial-fixation member and the baffle.

The device according to any of the preceding, wherein the proximal end of the anterior side of the atrial-fixation member is offset vertically from the proximal end of the posterior side of the atrial-fixation member.

The device according to any of the preceding, wherein the atrial-fixation member has an asymmetric shape with a length of the anterior side being longer than a length of the posterior side of the atrial-fixation member such that the anterior side of the atrial-fixation member is taller in a vertical direction than the posterior side of the atrial-fixation member.

The device according to any of the preceding, wherein the posterior side of the atrial-fixation member is stiffer than the anterior side of the atrial-fixation member. The atrial-fixation member may include a plurality of interconnected struts forming plural cells, with the struts on the posterior side of the atrial-fixation member being at least one of thicker, wider and/or having narrower gaps between adjacent struts than the struts on the anterior side of the atrial-fixation member.

Also disclosed is a method for repairing a regurgitant cardiac valve in a heart having an atrium having atrial walls, a ventricle having ventricular walls, a cardiac valve having at least two leaflets which have an open position and a closed position, the cardiac valve located at the boundary between the atrium and the ventricle, and an annulus surrounding the cardiac valve, the method comprising: providing a prosthetic leaflet device, including: an atrial-fixation member formed of a stent-like material, the atrial-fixation member defining a central lumen configured to fluidically couple the atrium and the ventricle, the atrial-fixation member having an anterior portion, a posterior portion, a proximal end, a distal end, a medial side, and a lateral side; and a baffle depending from a distal end of the posterior portion of the atrial-fixation member, an anterior portion of the baffle having a smooth, atraumatic surface which acts as a new coaptation surface for one of the two or more leaflets, a posterior portion of the baffle configured to engage and immobilize at least a portion of another of the two or more leaflets, the baffle protruding into the central lumen and approximating the closed position for the immobilized leaflet; and implanting the prosthetic leaflet device in the atrium such that the anterior, medial and lateral portions of the atrial-fixation member are spaced away from the annulus; and positioning the baffle to abut one of the at least two leaflets.

In the preceding method, the atrial-fixation member of the prosthetic leaflet device may include at least two suture loops having an eyelet, a suture strand threaded through the eyelets, wherein cinching the suture strand collapses the atrial-fixation member; and the step of implanting the leaflet prosthetic includes the steps of: cinching the suture strand to collapse the atrial-fixation member; placing the prosthetic leaflet device with the atrial-fixation member collapsed in the atrium; and uncinching the prosthetic leaflet device such that the atrial-fixation member expands into contact with the atrial walls.

In the preceding method, the prosthetic leaflet device may be fixed relative to the cardiac valve solely by the atrial-fixation member and the baffle.

Also disclosed is a heart valve repair device, comprising: an atrial fixation member defining a central lumen, the atrial fixation member; a baffle attached to the atrial fixation member, the baffle having an anterior portion with a smooth, atraumatic surface defining a prosthetic coaptation surface configured to coapt with at least one native leaflet of a native heart valve, and a posterior portion configured to engage and restrain at least a portion of a functionally deficient leaflet, the baffle extending radially inward into the central lumen to approximate a closed position of the functionally deficient leaflet. The device may further comprise a fabric covering at least partially surrounding the baffle. The baffle may comprise a biocompatible foam. The device may further comprise a plurality of frictional elements provided on the semi-circular ring and on a posterior portion of the baffle. The baffle may form a basket with a hollow interior. The baffle may comprise a plurality of anterior struts and a plurality of posterior struts with gaps interposed between adjacent ones of the anterior and posterior struts. The basket may have a mouth, the basket being covered by a fabric, with the mouth of the basket being uncovered. The anterior portion of the baffle may have a convex shape. The posterior portion of the baffle is sized and configured to engage with a central scallop of an anterior or a posterior mitral valve leaflet having three scallops, while leaving the remaining two scallops mobile. Alternatively, the posterior portion of the baffle is sized and configured to engage with the entire valve leaflet. The atrial fixation member may comprise a semicircular ring extending in a first plane and the baffle extends in a second plane which is parallel and vertically offset from the first plane. The anterior portion of the baffle comprises a biocompatible foam. The atrial fixation member may include a semi-circular ring sized to skirt the periphery of the annulus between the anterolateral commissure and a posteromedial commissure.

Also disclosed is heart valve repair device, comprising: an atrial fixation member defining a central lumen; at least one fixation mechanism having a trigonal anchor system and posterior hook; wherein the trigonal anchor system comprises a first trigonal extension attached to the first end of the partial ring and extending away from the baffle, a second trigonal extension attached to the second end of the partial ring and extending away from the baffle, and one of an anchor and an atraumatic tip attached to a terminal end of the first and second trigonal extensions; wherein the posterior hook is attached to a posterior portion of the atrial fixation member, and the posterior hook has a first portion which extends distally and a second portion which curves in a posterior direction, whereby the posterior hook is configured to extend into the ventricle and engage a ventricular side of a native cardiac annulus; and a baffle attached to the partial ring, the baffle having an anterior portion with a smooth, atraumatic surface which defines a prosthetic coaptation surface for one or more native leaflets, and a posterior portion configured to engage and displace at least a portion of a functionally deficient leaflet, the baffle extending into the central lumen and approximating a closed position for the displaced leaflet.

The preceding device, further comprising a fabric covering at least partially surrounding the baffle. The preceding device, wherein the baffle comprises a biocompatible foam. The preceding device, further comprising a plurality of frictional elements provided on the atrial fixation member and on a posterior portion of the baffle. In any of the preceding devices, the baffle may be a basket with a hollow interior. The baffle comprises a plurality of anterior struts and a plurality of posterior struts with gaps interposed between adjacent ones of the anterior and posterior struts. The basket has a mouth, the basket being covered by a fabric, with the mouth of the basket being uncovered.

In any of the preceding devices, the anterior portion of the baffle may have a convex shape. The posterior portion of the baffle is sized and configured to engage with and immobilize a central scallop of an anterior or a posterior mitral valve leaflet having three scallops, while leaving the remaining two scallops mobile. The posterior portion of the baffle is sized and configured to engage with the entire valve leaflet. The atrial fixation member includes a semi-circular ring sized to skirt the periphery of the native cardiac annulus between an anterolateral commissure and a posteromedial commissure.

Disclosed is a heart valve repair device, comprising: an atrial-fixation member defining a central lumen, the atrial-fixation member; and a baffle attached to the atrial-fixation member, the baffle having an anterior portion with a smooth, atraumatic surface which defines a coaptation surface configured to engage one or more native leaflets, and a posterior portion configured to engage and displace at least a portion of a functionally deficient leaflet, the baffle extending into the central lumen to approximate a closed position for the functionally deficient leaflet.

The previously described device further comprising a fabric covering at least partially surrounding the baffle. The baffle may comprise a biocompatible foam. The device further comprising a plurality of frictional engagement elements provided on the atrial-fixation member and the posterior portion of the baffle. The baffle may be a basket with a hollow interior. The baffle comprises a plurality of anterior struts and a plurality of posterior struts with gaps interposed between adjacent ones of the anterior and posterior struts. The basket has a mouth, the basket being covered by a fabric, with the mouth of the basket being uncovered.

The previously described device wherein the anterior portion of the baffle has a convex shape. The posterior portion of the baffle is sized and configured to engage with and displace a central scallop of an anterior or a posterior mitral valve leaflet having three scallops, while leaving the remaining two scallops mobile. The atrial fixation member includes a partially-circular, frustoconical shaped member having first and second ends, and a brace extending between the first and second ends of the atrial-fixation member.

Any of the previously described devices further comprising at least one fixation mechanism selected from the group (trigonal anchor and posterior hook), wherein the trigonal anchor comprises a first trigonal extension attached to the first end of the atrial-fixation member and extending away from the baffle, a second trigonal extension attached to the second end of the atrial-fixation member and extending away from the baffle, and one of an anchor and an atraumatic tip attached to a terminal end of the first and second trigonal extensions.

An embodiment of the present invention is a heart valve repair device for repairing a mitral valve having an anterior leaflet and a posterior leaflet, comprising: an atrial-fixation member configured to have a collapsed configuration and an expanded configuration, the atrial-fixation member having an expandable ring-shaped mesh, and the atrial-fixation member being configured to contact tissue of an atrial wall upstream of a native valve annulus; and a baffle extending radially inwardly from the atrial-fixation member, the baffle having an outer portion configured to displace the posterior leaflet toward a ventricular wall and restrain the posterior leaflet in an open position, an inner portion having a coaptation surface radially inward of the outer portion, wherein the inner portion is spaced apart from the outer portion by a distance such that the coaptation surface is positioned at least proximate a closed position of the anterior leaflet. The atrial-fixation member may be configured to contact only atrial wall tissue above the native valve annulus. The heart valve repair device may further include a biocompatible covering on a surface of the baffle. The baffle may include posterior struts extending in a downstream direction from the atrial-fixation member and anterior struts projecting inwardly and upwardly from a downstream end of the posterior struts, and wherein the heart valve repair device further includes a covering attached to the posterior and anterior struts. The atrial-fixation member may include a plurality of struts, and wherein the heart valve repair device further includes a covering attached to the struts of the atrial-fixation member.

### Brief Description of the Drawings

FIG. 1 is a diagram of a mitral valve;
FIGs. 2A-2I are views of a prosthetic leaflet device in accordance with embodiments of the present technology;
FIGs. 3A-3E are views of a prosthetic leaflet device in accordance with embodiments of the present technology;
FIGs. 4A-4B are views of a prosthetic leaflet device in accordance with embodiments of the present technology;
FIGs. 5A-5B are views of a prosthetic leaflet device in accordance with embodiments of the present technology;
FIGs. 6A-6B are views of a prosthetic leaflet device in accordance with embodiments of the present technology;
FIGs. 7A-7C are views of a prosthetic leaflet device in accordance with embodiments of the present technology; and
FIGs. 8A-8B are views of a prosthetic leaflet device in accordance with embodiments of the present technology.

### Detailed Description

Disclosed are various embodiments for addressing a regurgitant or incompetent cardiac valve including but not limited to a mitral valve. Although several examples of prosthetic leaflet devices are disclosed, all share a common theme of displacing at least a portion of at least one valve leaflet and providing a prosthetic, atraumatic coaptation surface for the remaining valve leaflet(s). The prosthetic coaptation surface does not move like a native leaflet in that it does not shift between an open and a closed position. Instead, the prosthetic coaptation surface can be fixed in a position or have limited movement that mimics or approximates the closed position of the partially or completely displaced native leaflet. Several embodiments of valve repair devices are described herein with reference to the mitral valve with the understanding that the utility of the present technology is not limited to the mitral valve and may be used with other heart valves.

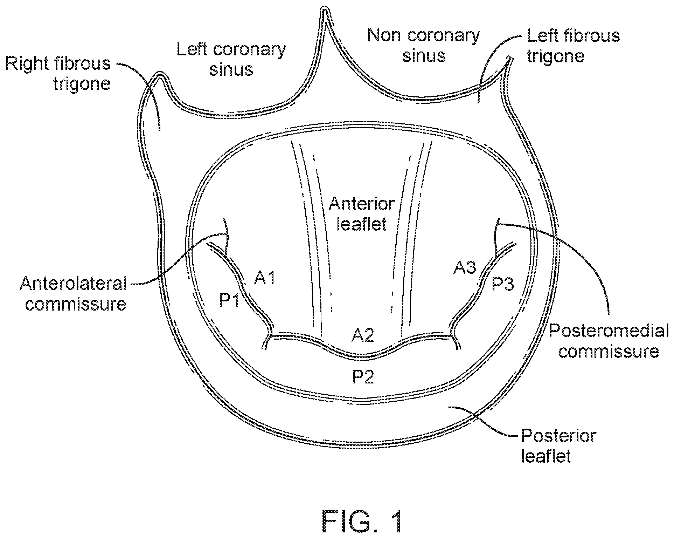

In the context of a mitral valve, the embodiments of the present technology may be used to displace and at least partially displace a portion of one of the leaflets while providing an atraumatic coaptation surface for the other native leaflet. Embodiments of the present technology displace a native leaflet with an implant which occludes approximately the same area as the fully closed native leaflet which is being replaced. The new coaptation surface mimics or approximates the closed position of the native leaflet. Referring to FIG. 1 which, illustrates a native mitral valve, the anterior and posterior leaflets of the mitral valve each have three well-defined scallops (A1-A3 and P1-P3). Some of the embodiments disclosed herein may displace and immobilize only one or two of the leaflet scallops while leaving the remaining leaflet scallops intact and mobile, but other embodiments can displace and immobilize an entire leaflet (i.e., all three scallops) while leaving the opposing leaflet intact and mobile. To simplify the description, several aspects of the present technology are described with respect to displacing and immobilizing the posterior leaflet of a mitral valve while providing an atraumatic coaptation surface for the anterior leaflet. However, the present technology could be used to displace and immobilize the anterior leaflet while providing an atraumatic coaptation surface for the posterior leaflet.

Referring still to FIG. 1, the mitral valve has an anterior leaflet and a posterior leaflet. The anterior leaflet has a semi-circular shape and attaches to two-fifths of the annular circumference. The motion of the anterior leaflet defines a boundary between the inflow (diastole) and outflow (systole) tracts of the left ventricle. The posterior leaflet of the mitral valve has a crescent shape and is attached to approximately three-fifths of the annular circumference. The posterior leaflet typically has two well-defined indentations which divide the leaflet into three individual scallops identified as **P1** (anterior or lateral scallop), **P2** (middle scallop), and **P3** (posterior or medial scallop). The three corresponding segments of the anterior leaflet are **A1** (anterior segment), **A2** (middle segment), and **A3** (posterior segment). The leaflet indentations aid in posterior leaflet opening during diastole.

The mitral valve has anterolateral and posteromedial commissures which define a distinct area where the anterior and posterior leaflets come together at their insertion into the annulus. The commissures may exist as well-defined leaflet segments, but more often this area is a subtle structure that can be identified using two anatomic landmarks: (a) the axis of corresponding papillary muscles, and (b) the commissural chordae, which have a specific fan-like configuration. Several millimeters of valvular tissue separate the free edge of the commissures from the annulus.

The mitral valve is an atrio-ventricular valve fluidically coupling the left atrium to the left ventricle. The mitral annulus defines the anatomical junction between the left ventricle and the left atrium. The fixed end of the leaflets is attached to the annulus. The anterior portion of the mitral annulus is attached to the fibrous trigones and is generally more developed than the posterior annulus. The right fibrous trigone is a dense junctional area between the mitral, tricuspid, non-coronary cusp of the aortic annuli and the membranous septum. The left fibrous trigone is situated at the junction of both left fibrous borders of the aortic and the mitral valve.

The mitral annulus is less well developed at the insertion site of the posterior leaflet as this segment is not attached to any fibrous structures and the fibrous skeleton in this region is discontinuous. The circumference of the posterior portion of the annulus may increase and lead to mitral regurgitation in association with left atrial or left ventricular dilation. The mitral annulus is saddle shaped, and during systole the commissural areas move in the direction of the atrium to make it more planar, while annular contraction also narrows the circumference. Both processes aid in leaflet coaptation and may be affected by processes such as annular dilatation and calcification. The mitral annulus is surrounded by several important anatomic structures, including the aortic valve, the coronary sinus, and the circumflex artery.

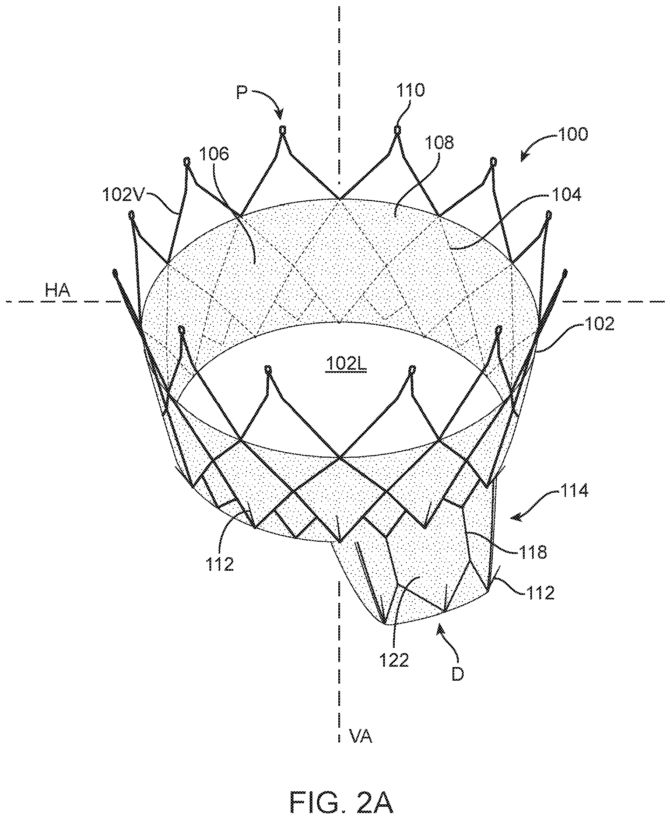

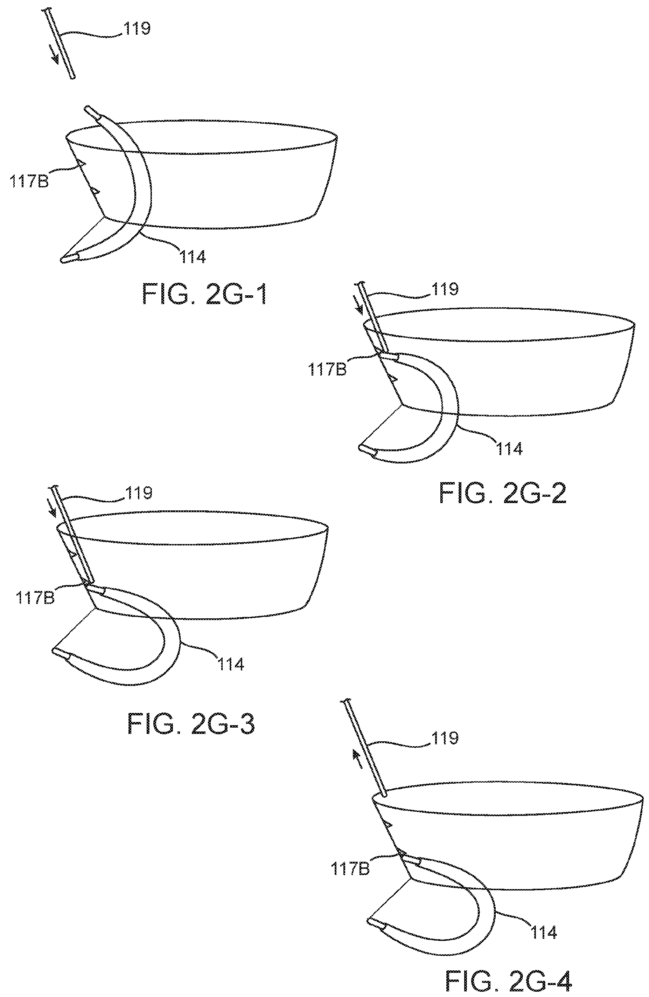

FIGs. **2A** - **2E** show a valve repair device in accordance with the present technology. The valve repair device can be a prosthetic leaflet device **100** having an atrial-fixation member **102** and a baffle **114** depending in a downstream direction from the atrial-fixation member **102.** The atrial-fixation member **102** can be configured to help position and hold the baffle **114** at a desired location with respect to the native valve anatomy, and the baffle **114** is configured to displace at least a portion of a native leaflet of the valve and create a prosthetic coaptation surface for at least a portion of one or more of the other leaflets of the native valve in the position provided by the atrial-fixation member **102.** The baffle **114,** for example, can be configured to displace a functionally deficient native leaflet, such as a posterior leaflet which is degenerated, torn, flailing, or otherwise no longer closing effectively during systole. In a valve with a dilated annulus such that regurgitant flow occurs between two native leaflets which no longer coapt effectively, the baffle **114** can extend beyond the dimensions of the existing leaflet to re-establish effective coaptation with the other leaflet(s).

The atrial-fixation member **102** can be formed of a mesh, such as a braid or stent-like structure, including a plurality of interconnected wires or struts **104** which cooperatively define a plurality of cells. The atrial-fixation member **102** aids in holding the baffle **114** in place by engaging the walls of the left atrium, and it may have a generally frustoconical shape. Each cell of the atrial-fixation member **102** defines an opening or through hole **106.** The unconstrained shape of the atrial-fixation member **102** may be generally circular or oval shaped. The atrial-fixation member **102** can have any number of through holes **106** or any shape or size such that the atrial-fixation member **102** contacts the left atrial wall with at least a threshold amount of radial force to stabilize the position of the baffle **114.** The radial force may be adjusted by varying the shape and dimensions of the struts (thickness, width, and spacing between struts) and the shape and dimensions of the through holes **106.** For example, wider and/or thicker struts **104** may increase the crush resistance, while larger openings or through holes **106** and/or thinner struts **104** may decrease the crush resistance. These concepts will be discussed in further detail below. In FIG. **2A****,** openings **106** are generally diamond-shaped.

The atrial-fixation member **102** may be formed of any biocompatible material such as stainless steel, a nickel-titanium alloy or a polymer. The atrial-fixation member **102** could be an elastic self-expanding material or a balloon-expandable material. According to a presently preferred embodiment, the atrial-fixation member **102** is formed of a super-elastic nickel-titanium alloy, e.g. Nitinol^{®}, that is self-expanding.

The prosthetic leaflet device **100** has a vertical axis **VA** in the direction of blood flow from the atrium to the ventricle and a horizontal axis **HA** orthogonal to the vertical axis. The prosthetic leaflet device **100** has a proximal side P and a distal side D. The prosthetic leaflet device has a proximal or leading end which faces the atrium and a distal or trailing end which faces the ventricle.

The atrial-fixation member **102** encircles or otherwise defines a lumen **102L** which when expanded spans a portion of the left atrium. The atrial-fixation member **102** may be covered in whole or in part by an optional biocompatible covering **108.** The biocompatible covering **108** may facilitate sealing and/or tissue ingrowth which may assist in long-term fixation of the device **100.** The optional biocompatible covering **108** on the atrial-fixation member **102** may be continuous with or without openings or windows, or it can be discontinuous with discrete sections such that gaps or uncovered portions of the atrial-fixation member **102** are interposed between covered portions. The biocompatible covering **108** may be a fabric formed of a polymer or biomaterial (Polyethylene terephthalate (PET), expanded polytetrafluoroethylene (ePTFE), silicone, urethane, pericardium, etc.). The covering **108** may be attached to the atrial-fixation member **102** by any conventional means including sutures, adhesives, sintering or the like. The covering **108** can be sutured to the inner surface of the atrial-fixation member **102,** i.e. the surface of the atrial-fixation member which faces the lumen **102L.** Alternatively, the covering **108** may be attached to the outer surface of the atrial-fixation member **102,** i.e., the side facing away from the lumen **102L,** which in use is the side facing the atrial wall.

The atrial-fixation member **102** is constructed and shaped to atraumatically provide sufficient fixation of the prosthetic leaflet device **100** in the left atrium and to enable delivery, positioning, and retrieval of the device. In the embodiment shown in FIGs. **2A****-2C,** the uppermost row of struts of the atrial-fixation member **102** form a plurality of inverted V-shaped structures or crown points (e.g., chevrons) **102V.** One or more of the chevrons **102V** may each include an eyelet **110** at the apex of the inverted V-shaped crown points. The eyelets **110** are sized to receive suture strands (not illustrated) used to deliver, orient and retrieve the prosthetic leaflet device **100** to/from the delivery catheter (not illustrated). The chevrons **102V** may extend generally vertically (parallel to the vertical axis **VA)** or they may be angled toward the lumen **102L** to minimize any trauma to the atrial wall, whereas the rest of the atrial-fixation member **102** diverges outwardly (e.g., away from the lumen **102L)** to have a frustoconical shape. The chevrons **102V** can be angled toward the lumen **102L.**

The prosthetic leaflet device **100** shown in FIGs. 2A-2E relies on atrial fixation to hold the device in place. The unconstrained size of atrial-fixation member **102** may be somewhat oversized relative to the size of the atrium in diastole to ensure that the prosthetic leaflet device **100** remains in its desired position. The atrial-fixation member **102** is configured to sit above the mitral valve annulus so that it does not affect the function of the other leaflets of the valve. The atrial-fixation member **102** may include a plurality of frictional engagement portions or cleats **112** which are adapted to frictionally engage, i.e., tent into the tissue without piercing into the tissue of the atrial wall. However, the cleats **112** may have sharpened tips or barbs configured to pierce into the atrial wall. The cleats **112** may be integrally formed with the struts **104** or otherwise attached to the struts **104.** The cleats **112** may extend (a) toward the eyelets **110** (i.e., opposite the direction of blood flow, such as upwards, proximally, towards the top of the atrium, or away from the ventricle in mitral valve applications), (b) laterally directly into the walls of the atrium, and/or (c) downwards away from the eyelets **110** (in the direction of blood flow, or downward, distally, toward the ventricle, or away from the atrium in mitral valve applications), or they may extend in multiple directions. The cleats **112** may be provided around the full perimeter of the atrial-fixation member **102,** or the cleats **112** may be provided only on portions of the atrial-fixation member **102.** For example, the cleats **112** may be provided on the anterior and posterior segments of the atrial-fixation member **102** and not on the lateral segments of the atrial-fixation member **102.** The prosthetic leaflet device **100** may rely on atrial fixation using the cleats **112** and/or oversizing of the atrial-fixation member **102** relative to the atrium to hold the prosthetic leaflet device **100** in place. The atrial-fixation member **102** is configured to sit slightly (e.g., 1-10mm) above the annulus such that, with exception of the baffle **114,** the prosthetic leaflet device **100** does not attach to the annulus.

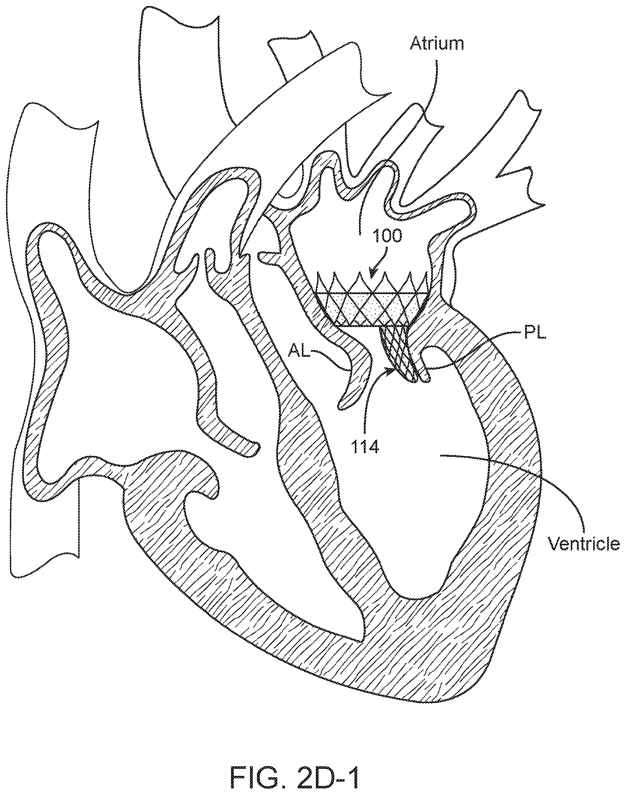

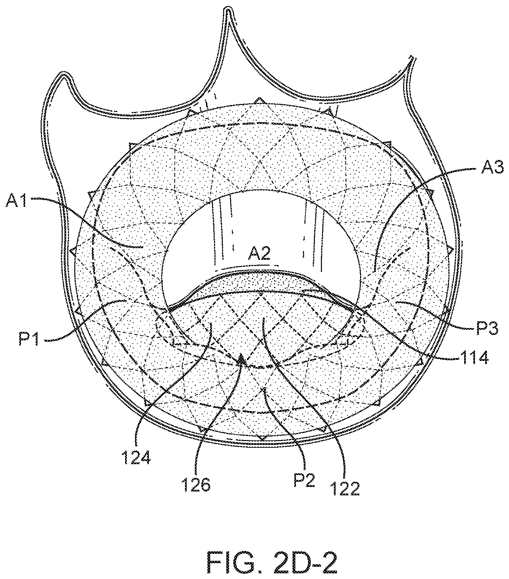

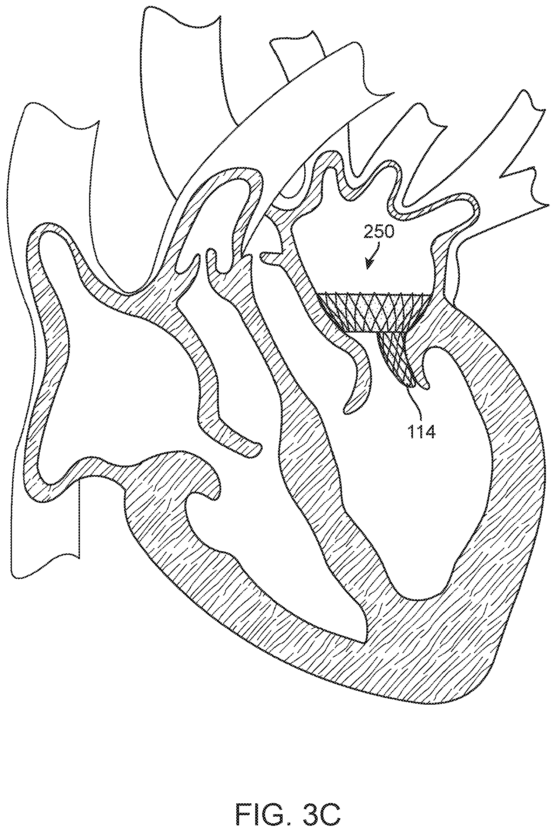

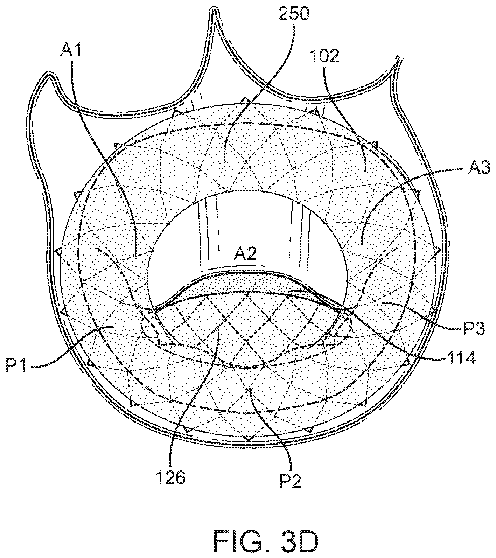

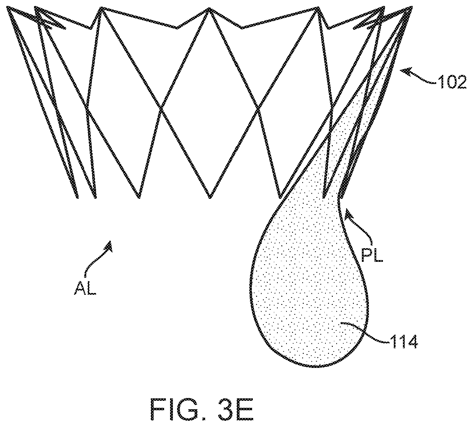

The baffle **114** extends distally (e.g., in the direction of blood flow) from the posterior portion of the atrial-fixation member **102.** The baffle **114** provides a prosthetic coaptation surface for the anterior leaflet, and it displaces a native leaflet which is damaged or otherwise does not sufficiently coapt with the anterior leaflet. See FIG. 2D-1. The baffle **114** can be shaped to provide these functions while enhancing or otherwise enabling coaptation with the other leaflets and enhancing blood flow around the baffle. The baffle **114** may, for example, have a teardrop shape, tapering as it extends upwards from a bulbous coaptation surface towards the posterior wall of the left atrium. (See, e.g., FIG. 3E)

The baffle **114** can be integral with the atrial-fixation member 102 such that it is manufactured from the same metal frame. The baffle **114** may alternatively be manufactured from a separate element and connected to a portion of the atrial-fixation member **102,** for example, by struts on the baffle **114** or the atrial-fixation member **102.**

In some embodiments, the baffle **114** can be formed of a biocompatible foam which is attached to the atrial-fixation member **102.** The baffle **114** can alternatively have an adjustable shape to enhance sealing with other leaflets, such as a baffle 114 comprising an inflatable bladder attached to atrial-fixation member **102.**

The baffle **114** can also contribute to fixation of the prosthetic leaflet device **100.** The posterior portion of the baffle **114** may include frictional elements, such as cleats **112,** configured to engage with the posterior leaflet and/or the annulus. The posterior portion of baffle **114** can also be shaped to engage the geometry of the annulus, ventricular wall, and atrial wall. For example, the posterior portion of baffle **114** can have a concave shape from the atrial end to the ventricular end, to engage the annulus and thereby prevent migration in an atrial or ventricular direction.

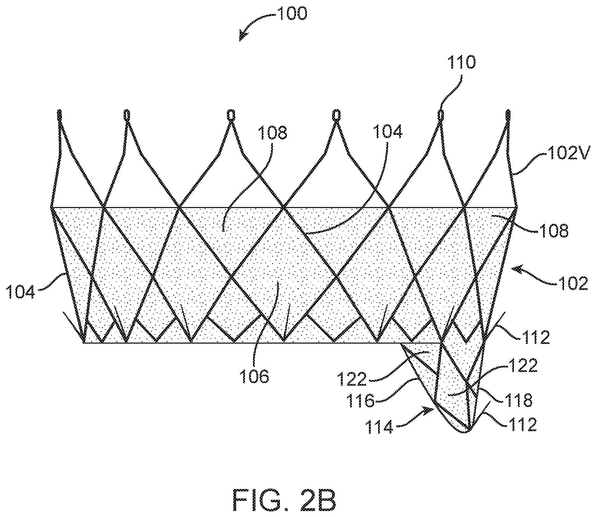

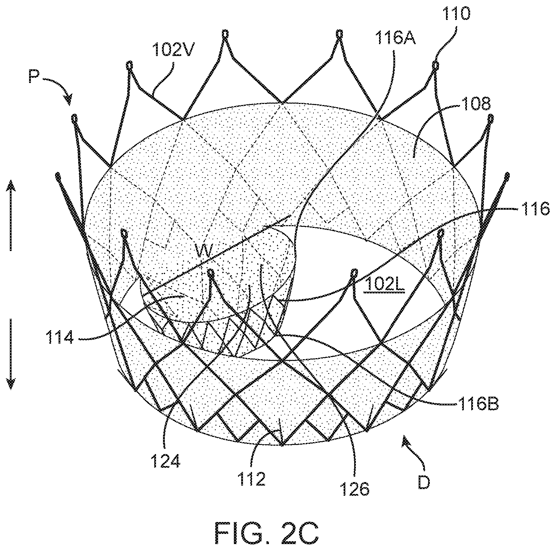

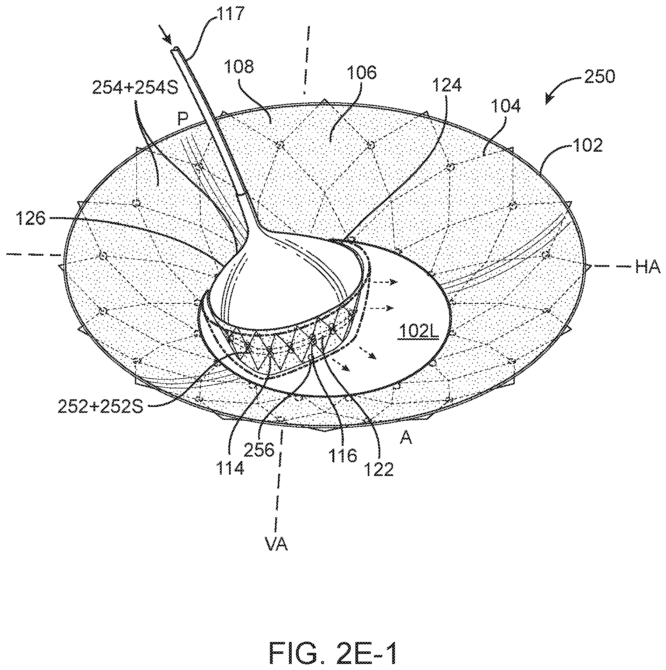

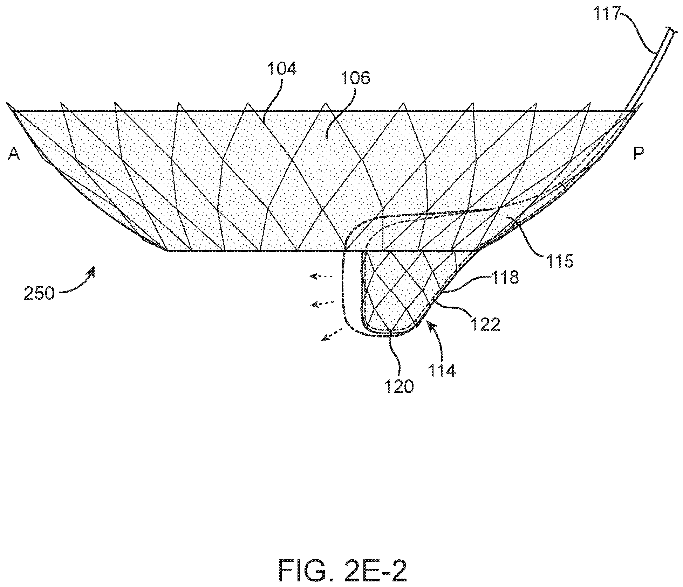

Referring to FIGs. 2A-2C, the baffle **114** can have a basket-like shape encompassing or surrounding a hollow interior. The basket-shaped baffle **114** may include anterior struts **116** (FIGs. 2B and 2C) connected to posterior struts **118** (FIGs. 2A and 2C) by a bridging portion **120** (FIG. 2E-2). A bio-compatible covering **122,** which may be the same material used for covering **108,** is attached to the baffle **114** to provide a fluid-tight seal with the adjacent leaflets of the valve. The anterior struts **116** may be connected to or integrally formed with the bridging portion **120** and the posterior struts **118,** for example from a single tubular mesh. In this embodiment, the baffle **114** is the only portion of the prosthetic leaflet device **100** which traverses the annulus and extends into the ventricle. See, **FIG. 2D.**

The baffle **114** may take a variety of shapes to facilitate coaptation with the posterior wall and adjacent leaflets and to ensure proper closure of the valve, optimize blood flow to prevent turbulent flow and/or formation of blood clots. The baffle **114** may also be configured to optimize stability of the baffle 114 throughout the cardiac cycle, and for ease of deployment and retrieval. In many embodiments, the anterior struts **116** protrude into the lumen **102L** and have a convex shape configured to provide an atraumatic coaptation surface for the anterior leaflet **AL.** (See, e.g., **FIG. 2E.)**

The anterior struts **116** span a width **W** (orthogonal to the vertical axis **VA)** which may be independent of the width spanned by the posterior struts **118.** The width spanned by the anterior struts **116** may be uniform along their length such that the width at the proximal end **116A** is equal to the width at the distal end **116B** as shown in **FIG.** 2C. However, in some embodiments the width spanned by the anterior struts **116** at the proximal end **116A** is slightly longer than the width at distal end **116B** forming wing-like portions which may facilitate sealing and coaptation. The anterior struts **116** are intended to provide an atraumatic coaptation surface for the anterior leaflet. As such, the anterior struts **116** preferably do not include any cleats **112.** Additionally, the width of the anterior struts **116** and/or the spacing between the struts may be adjusted to ensure an atraumatic coaptation structure for the anterior leaflet. In other words, the anterior struts **116** may be wider or more closely spaced than struts **104** and/or struts **118.**

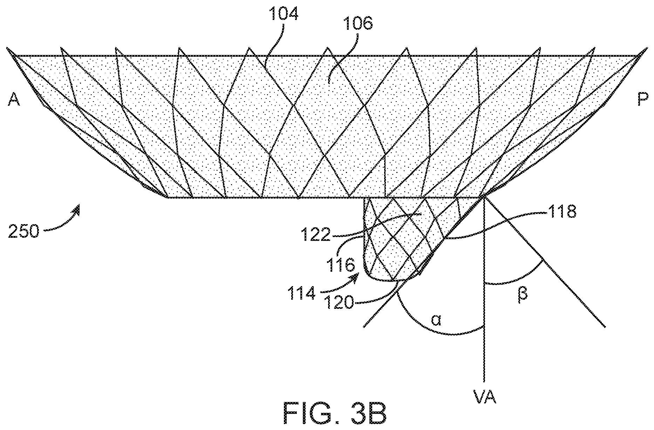

The posterior struts **118** may be generally parallel to the vertical axis **VA,** or they may have a somewhat arcuate shape which conforms with the ventricular wall. In other embodiments, the posterior struts **118** may extend at an angle toward the lumen **102L** to reduce contact with the ventricular wall and papillary muscles compared to parallel struts. The posterior struts **118** may extend at an angle alpha (shown in Figure 3B) ranging between **0** and **70** degrees relative to the vertical axis **VA** or at an angle beta ranging between 90 and 180 degrees relative to the vertical axis **VA** to reduce or increase contact with the ventricular wall. By reducing contact between the posterior struts **118** and the ventricular wall, the prosthetic leaflet device **100** may remain in a more constant position with respect to the native annulus. This is expected to provide more consistent coaptation between the prosthetic leaflet device **100** and the opposing native leaflet. Alternatively, providing a concave shape (an angle beta of less than 180 degrees between the atrial and ventricular portions of the posterior wall) may make it easier to position the device appropriately on the annulus and help to prevent device migration in the atrial or ventricular direction.

The posterior struts **118** may include cleats **112 (****FIG. 2B****)** which aid in fixation of the prosthetic leaflet device **100.** The cleats 112, for example, abut and engage with the posterior leaflet PL. In some embodiments, the cleats **112** may also engage with the posterior annulus and/or the ventricular wall. The cleats **112** at the ventricular end of the baffle **114** may be shorter to engage the thinner posterior leaflet tissue, whereas the cleats at the annular end of baffle **114** may be longer to engage the annular tissue.

The width W spanned by the posterior struts **118** may be configured to span the full width of the posterior leaflet including all three scallops **P1, P2** and **P3** (e.g., 25-55 mm). However, according to a presently preferred embodiment, the width spanned by the posterior struts **118** is less than the full width of the posterior leaflet PL. More particularly, the posterior struts **118** are configured to be somewhat wider than the width of the middle scallop **P2** (e.g., 20-35 mm) such that the struts **118** displace and at least partially immobilize only the middle scallop **P2** or engage only part of scallops **P1** and **P3.** This leaves scallops **P1** and **P3,** or at least a portion of scallops **P1** and **P3,** at least somewhat intact such that they are mobile and able to coapt with the baffle **114** and/or segments **A1** and **A3** of the anterior leaflet. (See dashed lines of the scallops **P1** and **P3 in** **FIG. 2D-2).** As a result, if the baffle **114** is slightly wider than the width of middle scallop P2, then the baffle **114** will engage all of scallop P2 and coapt with scallops P1 and P3 even if the prosthetic device **100** is slightly rotationally misaligned with the mitral valve.

In this embodiment, the anterior and posterior struts **116, 118** cooperatively define a basket or pocket **124 (****FIG. 2C****)** having a hollow interior volume and an opening or mouth **126 (****FIG. 2C****)** facing the eyelets **110** (proximal, atrial direction). (See also FIG. **2D-2).** Aside from the opening **126,** the basket **124** is sealed on all sides by the covering **122.** As described above, the fabric covering **108** on the atrial-fixation member **102** is completely optional and moreover may be discontinuous because it is used for tissue ingrowth or protection as opposed to providing a fluid tight seal. In contrast, the fabric covering **122** on the baffle **114** is intended to provide a fluid tight seal with the ventricular wall and the other leaflets of the valve. The shape of the basket **124** is configured to promote the circulation of blood through the hollow interior of the basket **124** and prevent the accumulation of stagnant blood thereby preventing the formation of thrombus. Additionally, the fabric covering **122** is preferably smooth to minimize any trauma to the anterior leaflet as the anterior leaflet coapts against the covering 122.

The covering **122** may be attached to the anterior and posterior struts **116, 118** by any conventional means including sutures, adhesives, sintering or the like. The covering **122** is intended to provide an atraumatic coaptation surface for the anterior leaflet and to prevent leakage of blood in a retrograde direction during systole. The covering **122** may be formed of the same material used for the covering **108** described previously.

The pocket **124** may also be inverted such that the mouth **126** faces downward towards the ventricle (distally), away from the eyelets 110. Still further, the mouth or opening **126** of the basket **124** may be sealed with the covering **122** thereby fully enclosing an interior volume of the pocket 124. This may be advantageous in ensuring non-turbulent blood flow and preventing the formation of thrombus.

The prosthetic leaflet device **100** is implanted in the atrium of the mitral valve such that the baffle **114** is the only portion of the device in contact with the annulus or which crosses the plane of the annulus. (See **FIG.** 2D). The baffle **114** may incidentally contact the annulus as it extends into the ventricle. In some embodiments, cleats **112** are provided on the posterior struts **118** to engage the annulus, but the prosthetic leaflet device **100** is not otherwise anchored to the annulus. More particularly, in a specific application the lower edge of the atrial-fixation member **102** is implanted 1-10mm, 1-8mm, 1-6mm, 1-4mm, 3-9mm, 3-6mm, 2mm, 3mm, 4mm, 5mm, 6mm, 7mm, 8mm, or 9mm above the annulus such that the atrial-fixation member **102** contacts only the wall of the atrium to allow the anterior leaflet to freely open and close under normal physiologic conditions. The atrial-fixation member **102** might be angled such that the anterior segment of the atrial-fixation member **102** is 10-30mm, 10-20mm, 10-15mm, 15-30mm 15-25mm, 15-20mm, 20-30mm, 10-25mm, 25-30mm, 10mm, 15mm, 20mm, 25mm, or 30mm above the anterior portion of the annulus or even positioned against the roof of the left atrium. As a result, the baffle **114** is the only portion of the prosthetic device **100** which extends into the left ventricle. (See FIG. **2D).**

In several embodiments, the prosthetic leaflet device **100** is retained in place by (a) the atrial-fixation member **102** pressing against the atrial wall and the frictional engagement of the cleats 112 with the atrial wall, and (b) the engagement of the baffle **114** and any cleats **112** on the baffle 114 against the posterior leaflet **PL** and the annulus. (See, e.g., **FIG. 2D-1.)** As such, it is advantageous for the atrial-fixation member **102** to bias the cleats 112 into engagement with the atrial wall. For example, the unconstrained size of the atrial-fixation member **102** may be slightly oversized relative to the diastolic size of the atrium, and the atrial-fixation member **102** may be formed of a super-elastic material such as Nitinol^{®} configured to exert a force biasing the cleats **112** into frictional engagement with the atrial wall (away from the lumen **102L).** If the atrial-fixation member **102** is formed of a balloon expandable material such as stainless steel, then the atrial-fixation member **102** is expanded to ensure that the cleats 112 engage with the atrial wall during atrial diastole as well as atrial systole.

The atrial-fixation member **102** may have an asymmetric shape with the proximal edge of the anterior portion of the atrial-fixation member **102** vertically displaced relative to the proximal edge of the posterior portion of the atrial-fixation member **102.** The height of the anterior portion of the atrial-fixation member **102** may additionally be taller or shorter than the posterior portion of the atrial-fixation member **102.**

The stiffness of the atrial-fixation member **102** may be non-uniform with discrete portions of the atrial-fixation member **102** being stiffer than others. For example, the anterior portion of the atrial-fixation member **102** may be more flexible than the posterior portion of the atrial-fixation member **102** or vice versa. There are many ways this may be accomplished. For example, the stiffness of a portion of the atrial-fixation member **102** may be adjusted by the making struts shorter, thicker, or wider, or by reducing the spacing between adjacent struts, such that one portion of the atrial-fixation member **102** is stiffer than another portion.

It may be desirable to modify or otherwise control the shape of the baffle **114** in situ to ensure coaptation and sealing of the anterior leaflet with the baffle. There are a number of ways in which this may be accomplished.

### Baffle including Inflatable Bladder

FIGs. 2E-1 through 2F-3 depict ways to adjust the size and/or shape of the baffle **114** in situ (in the body). FIGs. 2E-1 and 2E-2 show a bladder **115** inserted into the hollow interior **124** of the baffle **114.** The bladder **115** may be selectively inflated in situ with saline, blood, gel, or polymer via inflation lumen(s) **117** which extend outside of the body. Inflation of the bladder **115** modifies the shape of the baffle. Notably, inflation of the bladder **115** adjusts the amount the baffle **114** protrudes into the lumen **102L.** The anterior struts **116** may be configured to be less rigid than the posterior struts **118** to ensure that the anterior struts **116** preferentially deform inwardly toward axis VA-VA while the posterior struts **118** either do not deform or deform outwardly to a lesser extent than the anterior struts 116 deform inwardly. Inflating the bladder 115 deflects the anterior struts into the lumen **102L** and reduces the distance the anterior (opposing) leaflet must travel in order to coapt with the atraumatic surface of the baffle **114.** It may be useful for anterior struts **116** to be formed of stainless steel or the like to enable the bladder to plastically (permanently) reshape the anterior struts **116.**

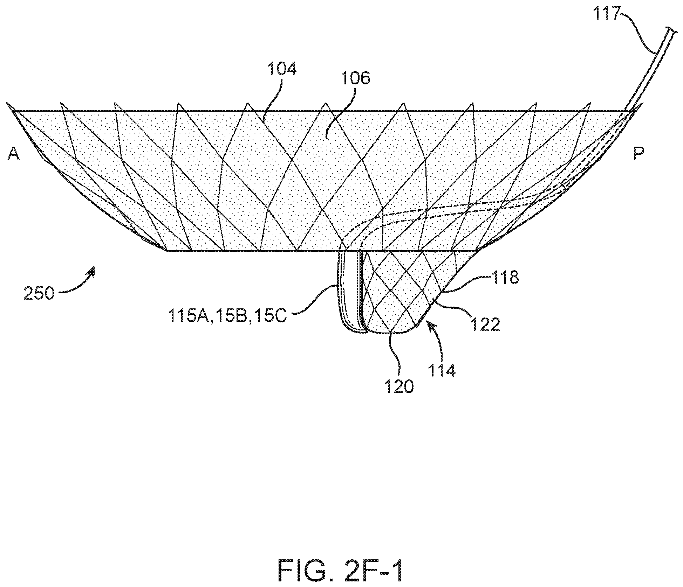

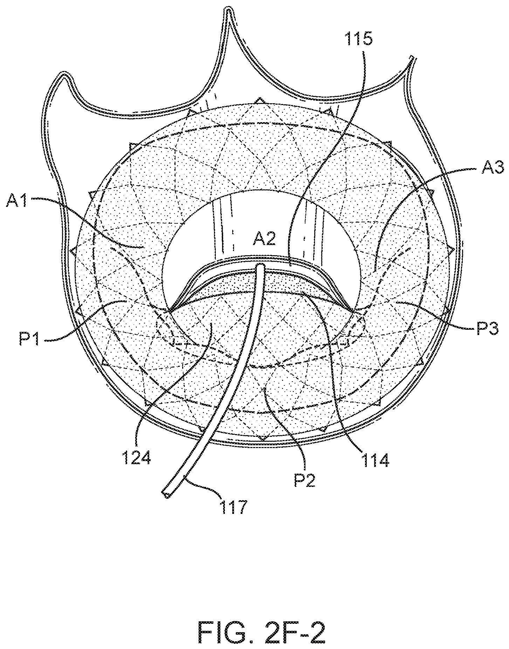

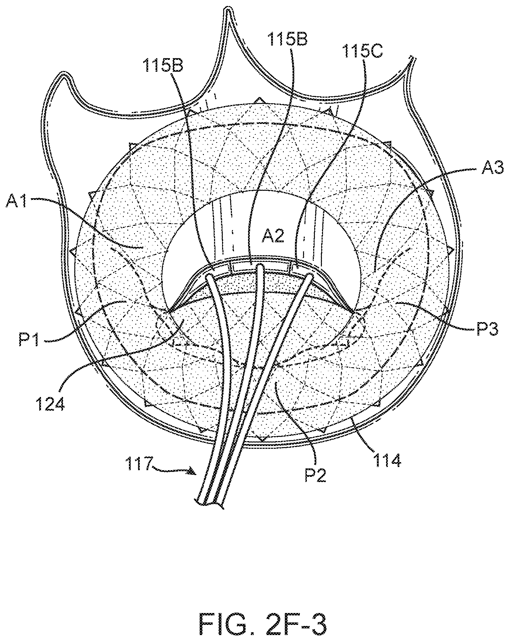

FIGs. 2F-1 through 2F-3 show another way to adjust the dimensions of the prosthetic coaptation surface by providing one or more bladders **115** (e.g., identified in FIG. 2F-1 as bladders **115A-C** are shown) on the anterior facing portion of the baffle **114.** A first bladder **115A** may be provided on a central portion of the anterior struts **116,** and it can be sized to coincide with the central lobe **A1** of the anterior leaflet. Alternatively, the first bladder **115A** may be sized to span the full width of the baffle 114 such that bladder coincides with all three lobes **A1, A2** and **A3.** Here, the first bladder **115A** serves as the atraumatic coaptation surface. Inflating the bladder modifies the amount the first bladder **115A** extends into the lumen **102L** thereby reducing the spacing between the baffle and that anterior leaflet.

FIG. 2F-3 shows device 250 with bladders **115A, 115B** and **115C** on the anterior struts 116 configured to coincide with scallops **A1,** A2 and A3 of the anterior leaflet. Each of the bladders **115A-C** can be inflated independently to further control the shape of the baffle **114.** As a result, the device **250** can be shaped in-situ to accommodate for rotational displacement of the device **250** with respect to the scallops, **A1, A2** and **A3** of the anterior leaflet or unique features of a specific anterior leaflet.

### Baffle with Adjustment Mechanism

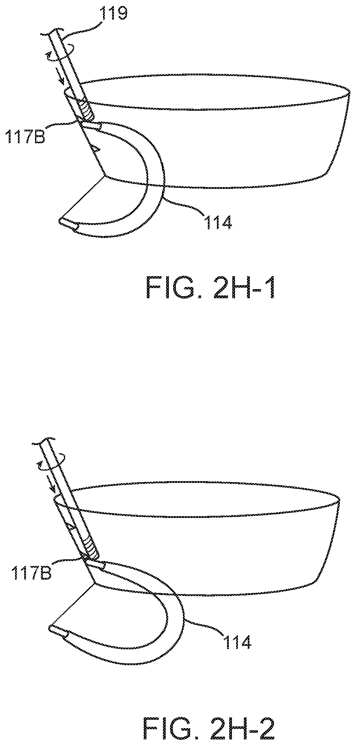

Actuating the shaft 119 adjusts the location of the proximal (atrial) connection between the baffle and the atrial fixation member, which in turn adjusts the amount that the baffle 114 extends into the lumen 102L.Another way to adjust the dimensions and shape of the baffle is to provide one or more adjustment mechanisms 117 such as a worm drive 117A (FIG. 2H) or ratchet 117B (FIGs. 2G-1 to 2G-3) operably connected to the baffle 114 and which are activated by a shaft 119 from outside of the body. FIGs. 2G-1 through 2G-3 show how the shape of the baffle 114 equipped with a ratchet 117B is adjusted by pushing on shaft 119. For example, as the shaft 119 is inserted into the ratchet 117B, the shaft 119 causes the baffle 114 to curve more. As a result, actuating the shaft 119 adjusts the location of the proximal (atrial) connection between the baffle and the atrial fixation member, which in turn adjusts the amount that the baffle 114 extends into the lumen 102L. A suture or cable (not illustrated) is operably connected to the ratchet release to facilitate unlocking the ratchet. The sides of the baffle 114 may be covered with a distensible material 122 such as PTFE or urethane to allow for expansion or contraction of the baffle 114.

FIG. 2H shows an embodiment of a leaflet prosthetic device having a worm drive **117A** adjustment mechanism for changing the shape of the baffle **114.** In operation, a shaft **119** with an engagement element at its distal end is engaged with the worm drive **117A.** The shaft **119** is rotated causing the worm drive **117A** to foreshorten. The foreshortening of the worm drive **117A** bends the baffle **114** such that the baffle **114** extends further into the lumen of the prosthetic leaflet device. As a result, the worm drive **117A** can adjust the baffle **114** to provide the desired amount of coaptation with the native valve leaflet(s).

### Prosthetic Leaflet Device with Skirt or Hem

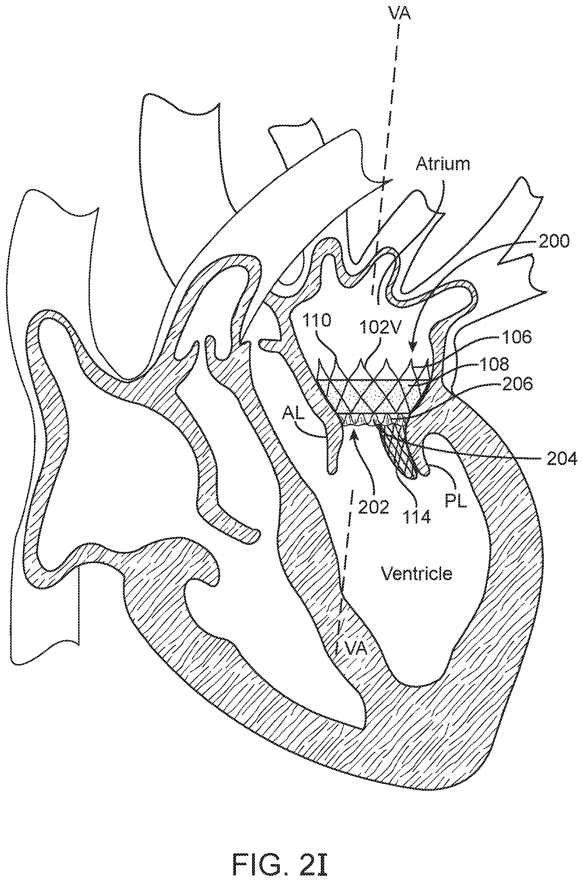

**FIG. 2I** shows a posterior prosthetic leaflet device **200** which is a slight variation of the posterior prosthetic leaflet device **100** shown above. Similar reference numbers refer to similar features. The prosthetic device **200** has an annular hem **202** which depends or extends in the direction of blood flow (e.g., distally or toward the annulus) from the distal end of the atrial-fixation member. The annular hem **202** includes a covering **204** and a structural reinforcement **206** which biases the covering **204** away from the lumen **102L,** i.e., towards the atrial wall. The structural reinforcement **206** may be connected to the prosthetic device **200** by the covering **204** and not otherwise attached to the prosthetic device **200.** The covering **204** may be integrally formed with or integrally connected to the covering **108, 122,** or the covering **204** may be attached to the atrial-fixation member **102.** The covering **204** is configured to promote tissue ingrowth and may be formed of the same or a different material than the covering **108.** The structural reinforcement **206** is intended to only provide annular (radial) support to the covering **204** and is specifically configured not to constrain vertical movement of the covering, i.e., movement parallel to the vertical axis **VA.** More particularly, structural reinforcement **206** biases the covering **204** away from the lumen **102L** (into the atrial wall). In use, the prosthetic leaflet device **200** is implanted in the left atrium such that the **hem 102** is slightly (0-10mm) above the mitral valve annulus.

### Prosthetic Leaflet Device without Chevrons

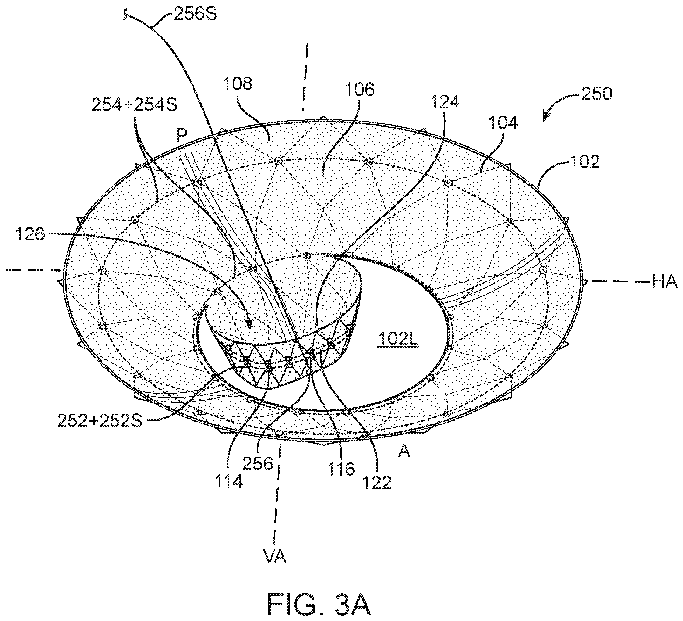

**FIGs. 3A****-3E** show a prosthetic leaflet device **250** which is a variation of the posterior prosthetic leaflet device **100.** Similar reference numbers refer to similar features. The prosthetic leaflet device 250 does not include the chevrons **102V** (FIGs. 2A and 2B) for delivering and positioning the prosthetic device 100. In place of the chevrons **102V** and the eyelets 110, prosthetic leaflet device **250** may include a first set of suture loops **252** at least partially spanning the periphery of the baffle 114, and a second set of suture loops **254** at least partially spanning the periphery of the atrial-fixation member 102. The first and second sets of suture loops **252, 254** include two or more suture loops. In the illustrated embodiment, the second set of loops **254** is provided proximate the distal end of the atrial-fixation member 102; however, the loops **254** may be positioned at any location on the atrial-fixation member **102** as desired. A third set of loops may be provided on the atrial-fixation member 102 if desired and may be cinched independently of suture loops **252** and **254.** The suture loops **252, 254** are configured to receive a filament (suture) **252S, 254S** or the like which may be used to selectively collapse the atrial-fixation member **102** and/or the baffle **114** by tightening the suture. The baffle **114** and atrial-fixation member **102** may be collapsed independently of one another. Collapsing the atrial-fixation member **102** and the baffle **114** facilitates repositioning and/or re-sheathing and removal of the prosthetic leaflet device **250.**

One or more additional suture loops **256** may be provided on the baffle **114** and is/are configured to receive a filament (suture) **256S.** Suture loop(s) **256** are optional and may be used to compress the baffle **114** for repositioning or removal. Suture loops **256** may also be used to evert the prosthetic leaflet device **250** by pulling on suture **256S** as an alternative method to disengage the prosthetic leaflet device **250** from the annulus for repositioning and/or re-sheathing of the prosthetic device **250.** In the illustrated embodiment the third suture loop(s) **256** are provided on the anterior side of the baffle **114,** i.e., the side facing the lumen **102L.**

As best seen in FIG. **3B****,** the posterior struts **118** of the baffle **114** may extend at an angle alpha relative to the vertical axis. In other words, the baffle **114** may be angled inwardly away from the ventricular wall towards the lumen **102L** in the direction of blood flow. This configuration spaces the downstream end of the baffle **114** radially inward of the ventricular wall or at least reduces the force exerted by the ventricular wall against the baffle **114** compared to some embodiments of the baffle **114** described above with respect to FIGs. **2A****-2E.** This is expected to reduce movement of the prosthetic device 250.

Alternatively, the posterior side of the baffle **114** may include a small notch or other indentation (not illustrated) extending laterally at an elevation corresponding to the position of the native annulus on the baffle **114** to provide additional clearance for the annulus. Alternatively, the posterior side of the baffle 114 may be angled outwardly toward the ventricular wall (away from the lumen **102L)** to enhance the radial outward force exerted against the native annulus to aid in anchoring in the posterior sub-annular space.

In some embodiments, the baffle **114** is formed of a biocompatible foam. This foam may be compressible to enable delivery through a small catheter and be self-expanding to resume its desired shape after delivery. A portion or all of the foam baffle **114** may be further covered by a biocompatible material such as expanded polytetrafluoroethylene to enhance tissue ingrowth or atraumaticity of the coaptation surfaces. The baffle **114** may, for example, have a teardrop shape. (See, e.g., **FIG. 3E****.)** The foam baffle **114** may include struts, e.g., on a posterior portion thereof which are connected to a posterior portion of the atrial-fixation member. The posterior portion of the baffle **114** may include frictional elements such as cleats **112** configured to engage with the posterior leaflet and possibly the annulus.

### Prosthetic Leaflet Device with Partial Anterior Atrial-Fixation Member

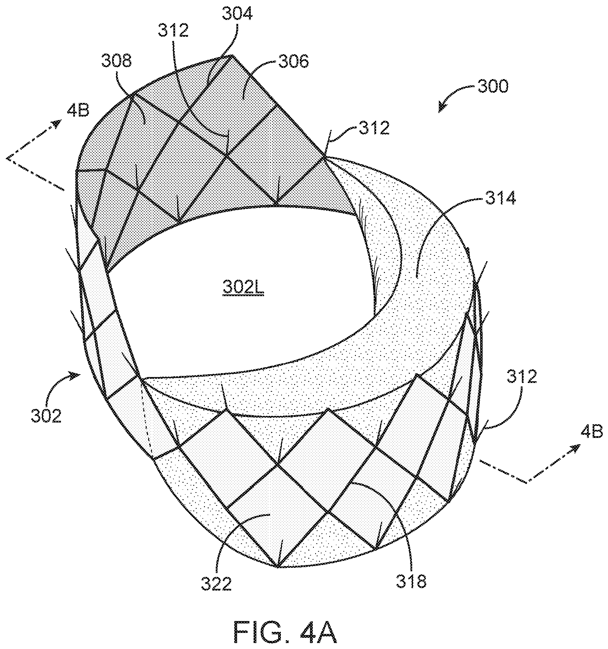

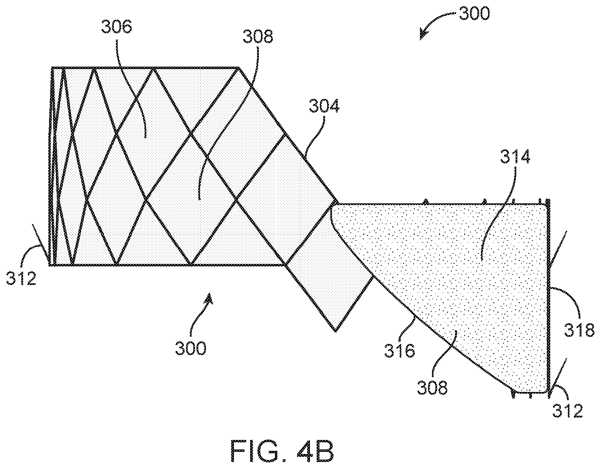

FIGs. 4A and 4B show a prosthetic leaflet device **300** having an atrial fixation member **302** configured to span part or all of the perimeter of the anterior leaflet (e.g., scallops **A1, A2** and **A3).** The atrial fixation member **302** can include struts **304** in a diamond pattern or another suitable pattern. Each cell of the atrial-fixation member **302** defines an opening or through hole **306.** The unconstrained shape of the atrial-fixation member **302** may be generally circular or oval shaped. The atrial-fixation member **302** can have any number of through holes **106** or any shape or size such that the atrial-fixation member **302** contacts the left atrial wall with at least a threshold amount of radial force to fix the position of the prosthetic leaflet device **300.** The radial force may be adjusted by varying the shape and dimensions of the struts (e.g., the thickness, width, and spacing between struts) and the shape and dimensions of the through holes **306.**

The atrial-fixation member **302** may be formed of any biocompatible material such as stainless steel, a nickel-titanium alloy or a polymer. The atrial-fixation member **302** could be an elastic self-expanding material or a balloon-expandable material. According to a presently preferred embodiment, the atrial-fixation member **302** is formed of a super-elastic nickel-titanium alloy, e.g. Nitinol^{®}, that is self-expanding.

The prosthetic leaflet device **300** further includes a baffle **314** attached to or integral with the atrial fixation member **302.** The atrial fixation member **302** and baffle **314** cooperatively define a lumen **302L** through which blood flows. The baffle **314** is configured to protrude into the lumen **302L** to provide an atraumatic coaptation surface for the anterior leaflet. In use, the prosthetic device **300** is implanted with the atrial fixation member **302** in the atrium and the baffle **314** extending from the atrium, across the annulus, and into the ventricle. All or part of the posterior leaflet is/are pushed out of the way by the baffle **314.** Several embodiments of the prosthetic device **300** are sized to coincide with the central lobe **P2** of the posterior leaflet without contacting at least a portion of the lobes **P1** and **P3,** such that lobes **P1** and **P3** remain mobile and can coapt with the baffle **314.** This may allow the baffle **314** to be smaller than the baffle **114** described above, which in turn is expected to reduce the forces exerted on the baffle **314** for enhancing long-term fixation of the prosthetic device **300.**

The atrial fixation member **302** and/or the baffle **314** may optionally include frictional elements such as cleats **312** adapted to frictionally engage (non-invasively) with the annulus, atrial wall, and/or posterior leaflet without penetrating (piercing) into the tissue. If desired, the cleats **312** may have sharpened ends configured to pierce into the tissue. In some embodiments, the cleats **312** are on the anterior portion of the atrial fixation member **302** and on the posterior portion of the baffle **314.** In a particular embodiment, the cleats **312** are not on the lateral sides of either the atrial fixation member **302** or the baffle **314.** The cleats **312** may engage the anterior atrial wall, posterior leaflet and/or the posterior annulus. The prosthetic leaflet device **300** may include additional anchoring elements such as hooks, barbs, screws, etc.

In some embodiments, the baffle **314** is formed of a biocompatible foam. The posterior portion of the baffle **314** may include frictional elements such as cleats **312** configured to engage with the posterior leaflet and possibly the annulus.

In other embodiments, the baffle **314** may be an open or closed basket-shaped baffle such as the one shown in FIG. 2B. The baffle **314,** for example, may be similar to the baffle **114** described above in FIGs. 2B and 2C such that the anterior struts **316** and posterior struts **318 (****FIG. 4B****)** define a portion of the baffle **314** and can be covered by a biocompatible covering **322** which may be the same material used for covering **108** described previously. The baffle **314** is configured to provide an atraumatic coaptation surface for the anterior leaflet in mitral applications. As described previously, the spacing, between struts **316** and the width of the struts **316** may be varied to ensure an atraumatic coaptation surface for the anterior leaflet.

### Trigonal anchor and/or Posterior Hook

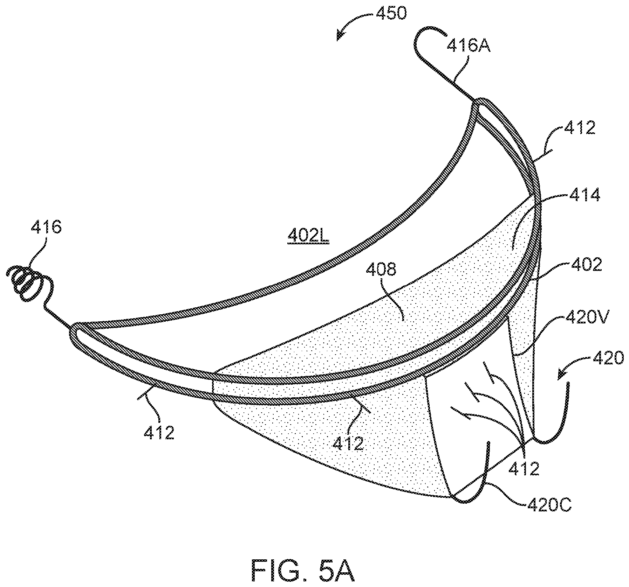

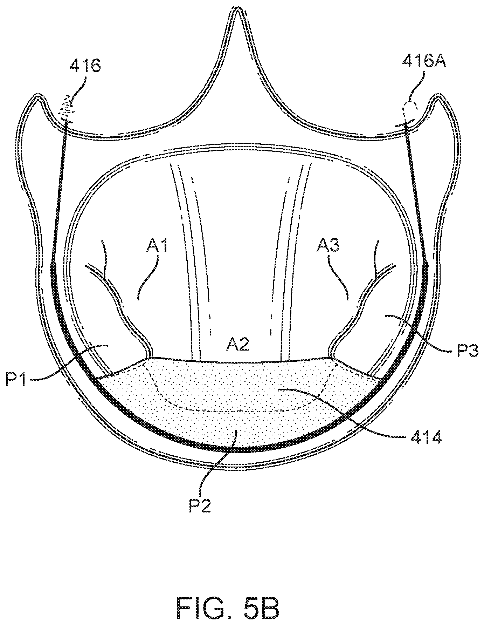

FIGs. 5A-5B Illustrate another prosthetic leaflet device **450** which is a variation of the prosthetic device **300.** The prosthetic device **450** includes a fixation ring **402** (e.g., a partially circular ring, a partially oval ring, etc.) configured to span the perimeter of part or all of the posterior leaflet (scallops **P1, P2** and **P3),** i.e., up to the leaflet commissures. The prosthetic leaflet device **350** replaces the atrial-fixation member **302** of prosthetic device **300** with one or more trigonal extensions **416, 416A.** The trigonal extensions **416, 416A** extend to the fibrous trigones (FIG. 1). To avoid impinging on the anterior leaflet, the trigonal extension may be offset vertically (proximally - toward the atrium and away from the annulus) and/or may be curved so as to skirt the perimeter of the annulus. The prosthetic device **450** may optionally include cleats 412 on the baffle and/or on the ring 402. FIG. **5A** depicts the prosthetic device **350** with both trigonal extensions and posterior hooks, but the illustrated embodiment is not intended to be limiting, such that each of these features could be separately incorporated into the prosthetic device **450** either in addition to or instead of the anterior atrial-fixation member and the posterior cleats. The trigonal extensions **416, 416A** are adapted to engage the fibrous trigones. The trigonal extension **416** has an anchor at the distal end of the extension configured to pierce into the fibrous trigone, whereas the trigonal extension **416A** has an atraumatic (curved or blunt) end adapted to non-invasively engage with the fibrous trigone. For the sake of illustration, the prosthetic leaflet device shown in FIG. 5A has one each of the trigonal extensions **416** and **416A,** but in use the prosthetic leaflet device would normally include one set of common trigonal extensions **416** or one set of common trigonal extensions **416A.**

The trigonal extensions **416, 416A** may extend (parallel to the horizontal axis **HA)** from the ring **402** to the fibrous trigone. The trigonal extensions **416, 416A** may be straight or curved. To minimize disruption to the normal movement of the anterior leaflet, it may be desirable for the trigonal extensions **416, 416A** to have an arcuate shape to skirt the perimeter of the anterior annulus. Additionally, or alternatively, the trigonal extensions may extend somewhat vertically (proximally) from the ring **402** to avoid the anterior leaflet.

The prosthetic leaflet device **450** may optionally include one or more posterior hooks **420** attached to a posterior portion of the ring **402.** Although not separately illustrated, the posterior hook **420** may be provided in place of, or in addition to, the posterior cleats 412 which engage the posterior leaflet and/or the posterior annulus. The posterior hook **420** includes a generally straight portion **420V** extending distally in a generally vertical direction and a portion **420C** which curves in a posterior direction configured to atraumatically engage the ventricular side of the annulus, the posterior ventricular wall, and/or the edge of the posterior leaflet.

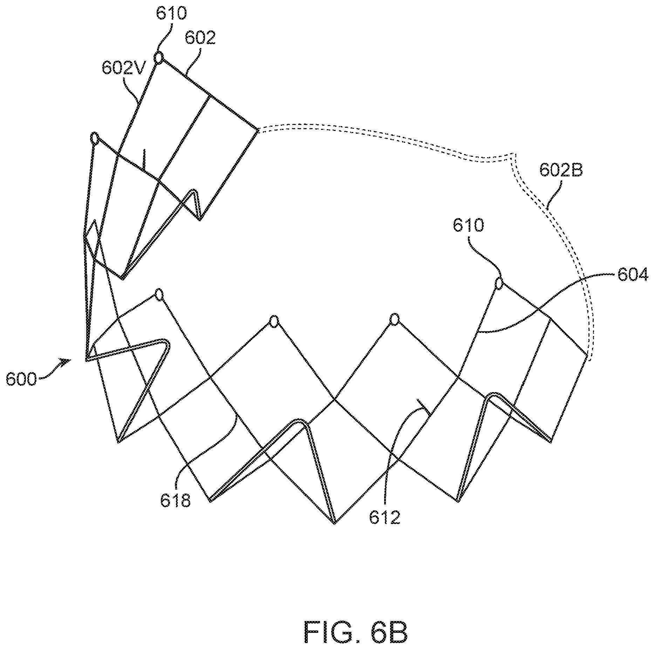

The prosthetic leaflet device **450** includes a baffle **414** attached to the distal partial ring **402** that protrudes into lumen **402L** and which provides an atraumatic coaptation surface for the anterior leaflet. In mitral valve applications, the prosthetic device **350** is implanted with the trigonal extensions **416, 416A** at the level of the annulus such that the baffle **414** extends into the ventricle. The baffle **414** displaces all or part of the posterior leaflet out of the way. According to several embodiments, the prosthetic device **350** is sized to coincide with the central lobe **P2** of the posterior leaflet, such that lobes **P1** and **P3** are mobile and able to coapt with the baffle **414.** See FIG. 6B.

In some embodiments, the baffle **414** is formed of a biocompatible foam. The baffle **414** may, for example, have a teardrop shape. In other embodiments, the baffle **414** has a basket shape enclosing a hollow interior volume as shown and described with reference to FIG. 2B. Thus, the basket-shaped baffle **414** may include a plurality of struts covered by a biocompatible covering **408** which may be the same material used for covering **108** described previously. The baffle **414** is configured to block regurgitant blood flow and to provide an atraumatic coaptation surface for the anterior leaflet.

### Prosthetic Leaflet Device with Posterior Atrial-Fixation Member

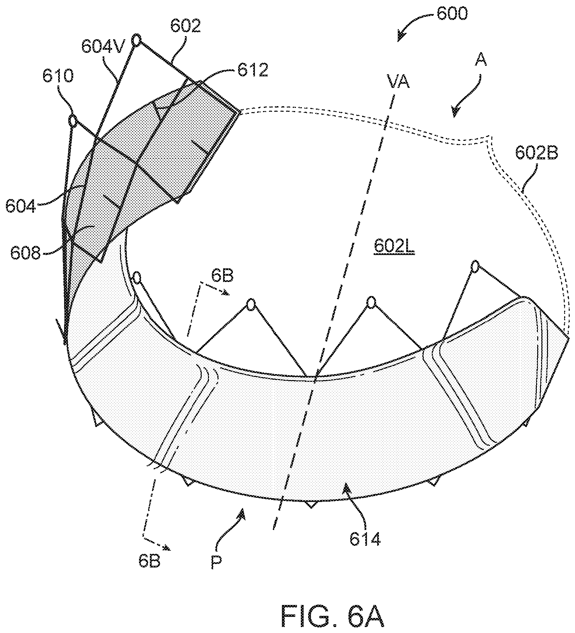

**FIGs. 6A****-6B** show a prosthetic leaflet device **600** including a fixation ring member **602** formed of a mesh, such as a braid or stent-like structure, having wires or struts **604** defining a plurality of cells where each cell has a through hole or opening **606.** For clarity, the prosthetic leaflet device 600 is shown inverted for a mitral valve application such downstream end is above the upstream end. The size, shape and number of the openings **606** are selected to optimize the radial force to ensure fixation. An optional bridging element **602B** (shown in phantom) may connect the ends of the fixation ring member **602** together such that the bridging element **602B** and the fixation ring member **602** cooperatively define a lumen **602L** which in operation fluidically couples the atrium and the ventricle. The ring segment **602** and the bridging element **602B** may be formed of any biocompatible material, including a super-elastic self-expanding material such a nickel-titanium alloy or a balloon-expandable material such as stainless steel. For example, the ring segment **602** and the bridging element **602B** are formed of a nickel-titanium alloy.

The prosthetic device **600** has a vertical axis **VA** in the direction of blood flow from the atrium to the ventricle and a horizontal axis orthogonal to the vertical axis. The prosthetic leaflet device **600** has an anterior side and a posterior side.

The ring segment **602** includes a plurality of baffle supports (struts) **613** (FIG. 6B) attached to or integrally formed with the ring **602.** The prosthetic device 600 further includes a baffle **614** attached to struts **613.**

In some embodiments, the baffle **614** is formed of a biocompatible foam. The baffle **614** may, for example, have a teardrop shape. The posterior portion of the baffle **614** and/or the struts **613** may include a plurality of frictional engagement portions or cleats **612** configured to engage with the posterior leaflet and possibly the annulus.

In other embodiments, the baffle **614** may have a basket shape (such as the baffle **114** shown in FIG. 3A) with a biocompatible covering **608** attached to the struts **618** to form a convex atraumatic surface against which the anterior leaflet may coapt. The baffle **614** protrudes into lumen **602L** such that a portion of the baffle **614** approximates the closed position of the posterior leaflet. The smoothness of the baffle, width of the struts **618,** and spacing between the struts **618** all contribute to providing an atraumatic coaptation surface.

The biocompatible covering **608** may be a fabric formed of a polymer or biomaterial (Polyethylene terephthalate (PET), expanded polytetrafluoroethylene (ePTFE), silicone, urethane, pericardium, etc.). The covering **608** may be attached to the ring segment **602** by any conventional means including sutures, adhesives, sintering or the like. In some embodiments the covering **608** is sutured to the struts **618.**

The uppermost row of struts **604** of the fixation ring **602** located at the most upstream end of the prosthetic device **600** form a plurality of inverted V-shaped structures or crown points or chevrons **604V.** Some, but not necessarily all, of the chevrons **604V** include an eyelet **610** at the apex of the V. The eyelets **610** are sized to receive suture strands (not illustrated) used to deliver, orient and retrieve the prosthetic leaflet device **600** to/from the delivery catheter (not illustrated). The chevrons **604V** may extend generally vertically (parallel to the vertical axis **VA)** or they may be angled toward the lumen **602L.**

The fixation ring **602** and the bridging portion **602B** may include a plurality of frictional engagement elements or cleats **612** which are adapted to frictionally engage the walls of the atrium. In some embodiments, the cleats **612** may have a sharpened end configured to pierce into the tissue of the atrial wall. The fixation ring **602** is configured to straddle the posterior mitral valve annulus with part of the ring in the atrium and part in the ventricle. The bridging portion **602B** is configured to skirt the perimeter of the anterior annulus or possibly extend from the anterior commissure to the posterior commissure to avoid interfering with the anterior leaflet and does not extend into the ventricle. The cleats **612** may be integrally formed with or integrally attached to the struts **604** and may extend upstream toward the eyelets **610** (e.g., proximally, away from the ventricle in mitral valve applications in mitral valve applications) and/or downstream away from the eyelets **610** (e.g., distally, toward the ventricle), or they may face in both directions. The cleats **612** are integrally formed with the struts and extend proximally toward the eyelets **610.**

### Prosthetic Leaflet Device with Posterior Hook

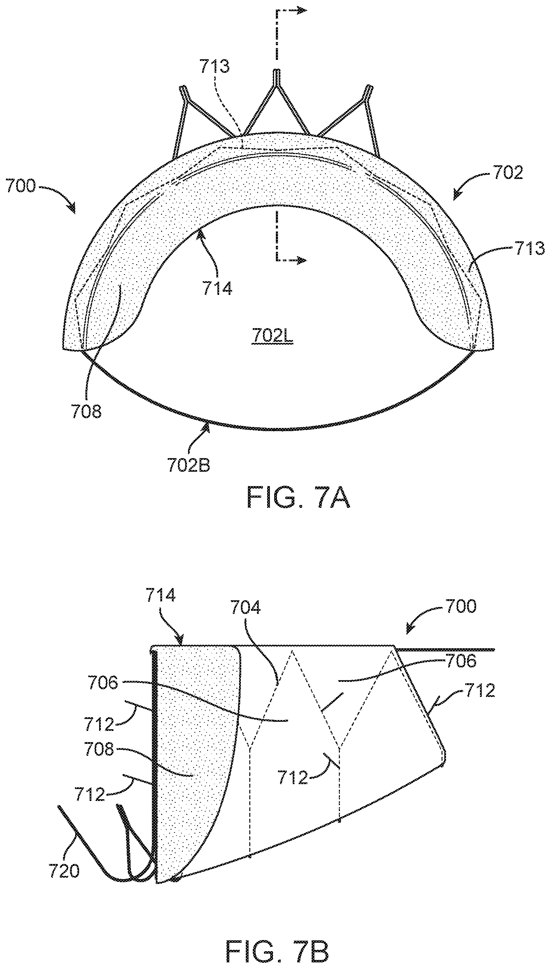

FIGs. 7A-7C depict a prosthetic leaflet device **700** including a fixation ring segment **702** formed of a mesh or stent-like material having a plurality of struts **704** defining a plurality of cells where each cell has a through-hole or opening **706.** The size, shape and number of the openings **706** can be configured to provide sufficient fixation of the prosthetic device **700** without exerting too much force against the tissue. The prosthetic device **700** can further include a bridging element **702B** that connects the ends of the ring segment **702** together such that the bridging element **702B** and the ring segment **702** cooperatively define a lumen **702L** which in operation fluidically couples the atrium and the ventricle. The ring segment **702** and the bridging element **702B** may be formed of any biocompatible material, including a super-elastic self-expanding material such a nickel-titanium alloy or a balloon-expandable material such as stainless steel. For example, the ring segment **702** and the bridging element **702B** are formed of a nickel-titanium alloy in a specific embodiment. The ring segment **702** includes a plurality of baffle supports (struts) **713** attached to or integrally formed with the ring segment **702.**

The prosthetic device **700** further includes a baffle **714** attached to the struts **713.** In some embodiments, the baffle **714** is formed of a biocompatible foam. The baffle **714** may, for example, have a teardrop shape. The posterior portion of the baffle **714** may include a plurality of frictional engagement portions or cleats **712** configured to engage with the posterior leaflet and possibly the annulus. In other embodiments, the baffle may have a basket shape (such as baffle **114** in FIG. **3A****)** with a biocompatible covering **708** attached to the supports **713** to form baffle **714** which provides an atraumatic surface against which the anterior leaflet may coapt. The biocompatible covering **708** may be a fabric formed of a polymer or biomaterial (Polyethylene terephthalate (PET), expanded polytetrafluoroethylene (ePTFE), silicone, urethane, pericardium, etc.). The covering **708** may be attached to the ring **702** by any conventional means including sutures, adhesives, sintering or the like. Thus, in use the baffle **714** replaces the posterior leaflet.

The ring segment **702** and the bridging portion **702B** may include a plurality of frictional engagement portions or cleats **712** which are adapted to frictionally engage the walls of the atrium. For example, the cleats **712** can "tent" into the tissue without piercing into the tissue of the atrial wall. The ring segment **702** is configured to straddle the posterior mitral valve annulus with part of the ring in the atrium and part in the ventricle. The bridging portion **702B** is configured to skirt the perimeter of the anterior annulus and does not extend into the ventricle. The cleats **712** may be integrally formed with or integrally attached to the struts **704,** and they may extend upstream (e.g., proximally, away from the ventricle) and/or downstream (e.g., distally, toward the ventricle), or they may face in both directions. In the illustrated embodiment, the cleats **712** are integrally formed with the struts and extend proximally (upward).

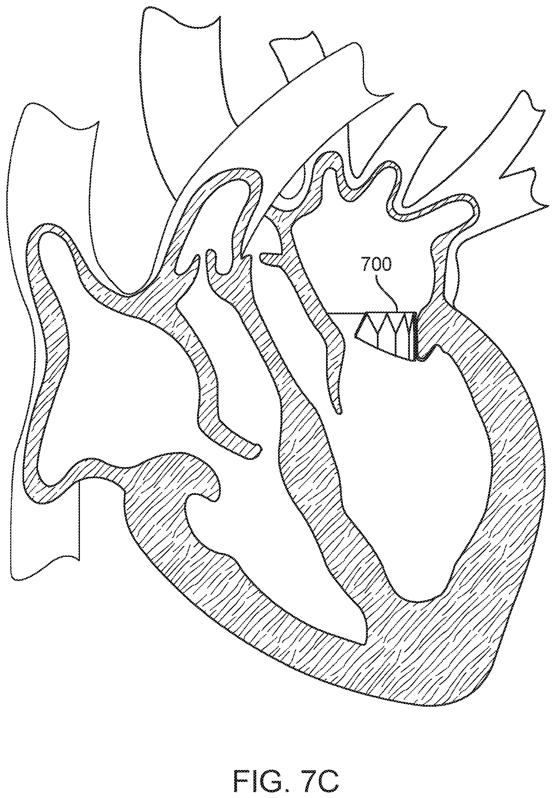

The prosthetic leaflet device **700** may optionally include a posterior hook **720** attached to a posterior portion of the ring segment **702.** The posterior hook **720** is configured to atraumatically engage the ventricular side of the annulus, the posterior ventricular wall, and/or free edge of the posterior leaflet. (See, **FIG.** 7C.). This is expected to further fix the prosthetic leaflet device **700** in place while also retaining the native posterior leaflet.

### Semi-Circular Prosthetic leaflet device

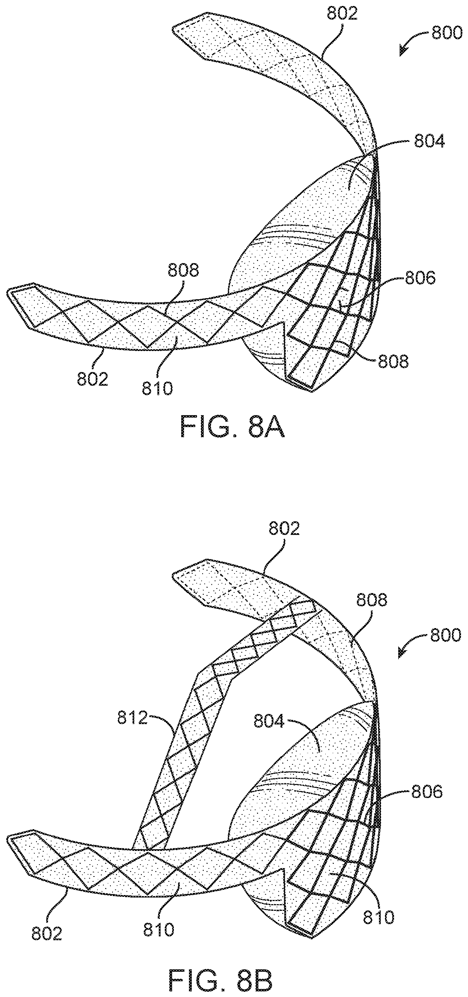

FIGs. **8A** and **8B** show a prosthetic leaflet device **800** including a pair of stabilizing arms **802** and a baffle **804** attached to the stabilizing arms **802.** The stabilizing arms **802** extend in an arc, such as a semi-circle or semi-oval, and are intended to extend along the posterior atrial wall from the anterolateral commissure to the posteromedial commissure. The stabilizing arms **802** are configured to frictionally engage with the atrial wall and may be formed of a balloon expandable material such as stainless steel or a self-expanding material such as a nickel-titanium alloy (e.g., Nitinol^{®}) having an unrestrained size which ensures engagement with the atrial wall during diastole as well as systole. The stabilizing arms **802** preferably include a plurality of cleats **806** adapted to engage tissue and assist in fixation of the prosthetic device **800.** The stabilizing arms **802** may optionally be covered with a biocompatible fabric covering **810** to promote tissue in-growth.

The baffle **804** is intended displace the posterior leaflet and provide an atraumatic coaptation surface for the anterior leaflet, thereby preventing or at least inhibiting regurgitant blood flow. In some embodiments, the baffle **804** is formed of a biocompatible foam. The baffle **804** may, for example, have a teardrop shape. The posterior portion of the baffle **804** may include a plurality of frictional engagement portions or cleats **806** configured to engage with the posterior leaflet and possibly the annulus. In other embodiments, the baffle **804** may have a basket shape (like baffle **114** in FIG. **3A****)** enclosing a hollow interior such as described with reference to FIG. 2B. The baffle **804** extends in a downstream direction (e.g., distally) from a posterior portion of the stabilizing arms **802** and includes a plurality of struts **808** forming a somewhat convex structure covered by biocompatible covering **810** having pores to facilitate tissue in-growth. The posterior side of the baffle **804** is configured to frictionally engage one or more lobes of the posterior leaflet. The cleats **806** on the posterior portion of the baffle are not obstructed by the covering **810.**

FIG. 8B shows the prosthetic leaflet device **800** with an optional strut **812** spanning the otherwise free ends of the stabilizing arm **802.** The strut **812** may help ensure that the stabilizing arms stay engaged with the atrial wall. The stabilizing arm **802** is configured to extend between the anterior and posterior leaflet commissure.

In use the prosthetic leaflet device **800** is implanted with the baffle **804** straddling the annulus with part of the baffle **804** extending into the ventricle and engaging with the posterior leaflet. The stabilizing arms **802** may remain in the atrium, skirting the periphery of the annulus.

### Deployment of the Prosthetic Leaflet Device

Each of the prosthetic leaflet devices disclosed herein may be delivered via a trans-septal, trans-atrial, or trans-apical approach. The implant may for example be delivered from a 3-9mm, 4-8mm, 5-7mm, 3mm, 4mm, 5mm, 6mm, 7mm, 8mm or 9mm inner diameter sheath with the sheath tip positioned approximately at P2. Controlled unsheathing allows the implant to expand radially in place against the annulus on the posterior side.

Some of the embodiments utilize control wires or sutures to collapse the prosthetic device for repositioning and/or re-sheathing in the catheter prior to final release and to allow for complete recovery of the implant. A circumferential cinching element may also be contained within the baffle support elements to facilitate a controlled radial expansion during deployment and to aid in radial contraction prior to complete recovery of the implant. In other embodiments, the control wire(s) and/or control arm(s) may bend the anterior portion of the atrial-fixation member inwards towards the posterior P2 portion of the atrial-fixation member upon recovery to reduce the overall diameter of the atrial-fixation member and ensure disengagement of the fictional elements from the tissue.

Several embodiments of implants in accordance with the present technology are delivered trans-septally. Prior to placement in the annulus, the prosthetic leaflet device is advanced partially out of the delivery catheter into the atrium, where it can be rotationally aligned with respect to the native valve and prepared for placement. The prosthetic leaflet device is advanced into the annulus and allowed to expand and resume its unconstrained shape, thereby engaging the annulus and the sub annular space posteriorly with frictional elements and then engaging the anterior surface of the left atrium with radial force. In one embodiment, the posterior aspect of the structural ring has an active (e.g., movable) hinge that folds radially inward during delivery and/or recovery.

In several embodiments the baffle is the first portion of the prosthetic leaflet device that is deployed. This enables the baffle to be positioned at a desired location and orientation with respect to the native valve, and in particular the posterior leaflet and the annulus in mitral valve applications. Then the posterior ventricular hook or cleats are engaged with the posterior leaflet and annulus. If the prosthetic leaflet device is equipped with trigonal extensions, then the trigonal extensions are the next portions of the prosthetic leaflet device that are deployed from the catheter. The structural ring and finally the atrial-fixation member (if present) are the last two portions of the prosthetic leaflet device to be deployed from the catheter into the left atrium.

This concept can also be applied to other valves besides the mitral valve. For example, some patients have aortic valve regurgitation due to degeneration of one of the leaflets or dilation of the aortic root. One embodiment of a device for repair of an aortic valve comprises a blocker which displaces the non-coronary cusp of the aortic valve. The ventricular surface of the blocker is shaped roughly like the ventricular surface of the non-coronary cusp in the closed position. It can be somewhat larger than the native leaflet, so that it coapts with the other leaflets of the aortic valve. The peripheral surface of the blocker can be shaped to generally conform to the shape of the non-coronary sinus. The surface which faces towards the aortic lumen can taper from the coaptation surfaces to the aortic wall 1-4 cm downstream (cranially) of the aortic valve. The blocker can be connected to a stent which is deployed in the aortic root or ascending aorta to hold it in place. As in the prior embodiments, the blocker can be formed from foam or an expanding metal or polymer framework which can be covered with expanded PTFE, pericardium, or other biocompatible tissue which forms an atraumatic coaptation surface for the other leaflets of the valve. The remaining two leaflets of the aortic valve, if they are not stenosed, should provide ample luminal area so the repaired valve does not have an excessive pressure gradient as blood flows through it.

## Claims

1. A heart valve repair device (100), comprising:
an atrial-fixation member (102) having an expandable mesh configured to have an oval or circular shape in a deployed configuration, the atrial-fixation member (102) defining a central lumen (102L) and being configured to contact an atrial wall upstream of an annulus of a native heart valve;
and
a baffle (114) extending from a portion of the atrial-fixation member (102), the baffle (114) having an anterior portion with an atraumatic surface configured to coapt with at least a portion of one or more native leaflets of the native heart valve, a posterior portion configured to engage and displace at least a portion of another native leaflet of the native heart valve, wherein the baffle (114) extends radially inward from the atrial-fixation member (102) into the central lumen to approximate a closed position of the displaced native leaflet.

2. The device of claim 1, wherein the baffle includes struts configured into a basket having a hollow interior volume, the baffle further including a restraining portion configured to engage and restrain at least a first portion of a functionally deficient native leaflet while leaving a second portion of the functionally deficient native leaflet mobile, the baffle extends radially inward into the central lumen to approximate a closed position for the functionally deficient leaflet.

3. The device according to claim 1, further comprising a plurality of frictional elements (112) provided on portions of the atrial-fixation member or the baffle; or
wherein the baffle comprises plurality of anterior struts (116) and a plurality of posterior struts (118) with gaps interposed between adjacent ones of the anterior and posterior struts.

4. The device according to claim 2, wherein the medial and lateral sides of the atrial-fixation member do not include any frictional elements.

5. The device according to claim 1, wherein the baffle comprises a basket enclosing a hollow interior; or wherein the baffle comprises a biocompatible foam.

6. The device according to claim 2, comprising:
a fabric covering (122) at least partially surrounding the baffle; and
the basket has a mouth which is covered by the fabric or which is not covered by the fabric.

7. The device according to claim 1, wherein the anterior portion of the baffle has a smooth surface and/or a convex shape; and/or
wherein the posterior portion of the baffle is sized and configured to engage with a central scallop a native mitral valve leaflet having three scallops, while leaving the remaining two scallops mobile.

8. The device according to claim 1, further comprising:
a first set of suture loops (252) circumferentially disposed around the baffle, each suture loop of the first set of suture loops having a lumen; and
a first suture (252S) disposed in the lumen of the suture loops of the first set of suture loops and interconnecting adjacent suture loops of the first set of suture loops;
wherein a diameter of the baffle is adjusted by cinching the first suture; and/or
further comprising:
a second set of suture loops (254) circumferentially disposed around the atrial-fixation member, each suture loop of the second set of suture loops having a lumen; and
a second suture (254S) disposed in the lumen of the suture loops of the second set of suture loops and interconnecting adjacent suture loops of the second set of suture loops;
wherein a diameter of the atrial-fixation member is adjusted by cinching the second suture; and/or
further comprising:
one or more third suture loops (256) provided on the baffle,
a third suture (256S) disposed in the lumen of the one or more third suture loops;
wherein the pulling on the third suture displaces the baffle.

9. The device according to claim 1, further comprising at least one fabric covering (108) attached to and at least partially spanning the atrial-fixation member, wherein the fabric covering facilitates tissue ingrowth.

10. The device according to claim 1, further comprising at least one fabric segment of fabric depending from the distal end of the atrial-fixation member; optionally,
further comprising a biasing member (206) attached to the at least one fabric segment and biasing the fabric segment away from the central lumen.

11. The device according to claim 1, wherein the atrial-fixation member comprises at least two rows of cells; or
wherein:
the atrial-fixation member further comprises a row of chevrons;
at least two of the chevrons include a through-hole; and
a suture threaded through the through-hole;
wherein cinching the suture adjusts a diameter of the atrial-fixation member.

12. The device according to claim 1, wherein the atrial-fixation member has a frustoconical shape; optionally,
wherein the atrial-fixation member is configured to engage solely with an atrial wall of the native heart valve.

13. The device according to claim 12, wherein a size of the atrial-fixation member unconstrained by external forces is larger than a size of a native atrium in diastole, optionally,
wherein the device is fixed relative to the native cardiac valve solely by the atrial-fixation member and the baffle.

14. The device according to claim 1, wherein the atrial fixation member has an anterior side and a posterior side, a proximal end of the anterior side of the atrial-fixation member is offset vertically from a proximal end of the posterior side of the atrial-fixation member, and/or
wherein the atrial-fixation member has an asymmetric shape with a length of an anterior side being longer than a length of a posterior side of the atrial-fixation member such that the anterior side of the atrial-fixation member is taller in a vertical direction than the posterior side of the atrial-fixation member.

15. The device according to claim 1, wherein a posterior side of the atrial-fixation member is stiffer than an anterior side of the atrial-fixation member; optionally,
wherein the atrial-fixation member includes a plurality of interconnected struts forming plural cells, with the struts on the posterior side of the atrial-fixation member being at least one of thicker, wider and/or having narrower gaps between adjacent struts than the struts on the anterior side of the atrial-fixation member.

## Patentansprüche

1. Herzklappenreparaturvorrichtung (100), umfassend:
ein Vorhoffixierungselement (102), das ein expandierbares Geflecht aufweist, das konfiguriert ist, um in einer entfalteten Konfiguration eine ovale oder kreisförmige Form aufzuweisen, wobei das Vorhoffixierungselement (102) ein zentrales Lumen (102L) definiert und konfiguriert ist, um eine Vorhofwand stromaufwärts eines Anulus einer nativen Herzklappe zu kontaktieren; und
ein Baffle (114), das sich von einem Abschnitt des Vorhoffixierungselements (102) erstreckt, wobei das Baffle (114) einen anterioren Abschnitt mit einer atraumatischen Oberfläche, die konfiguriert ist, um mit mindestens einem Abschnitt eines oder mehrerer nativer Segel der nativen Herzklappe zu koaptieren, und einen posterioren Abschnitt aufweist, der konfiguriert ist, um mit mindestens einem Abschnitt eines anderen nativen Segels der nativen Herzklappe in Eingriff zu kommen und diesen zu verschieben, wobei sich das Baffle (114) von dem Vorhoffixierungselement (102) radial nach innen in das zentrale Lumen erstreckt, um sich einer geschlossenen Position des verschobenen nativen Segels anzunähern.

2. Vorrichtung nach Anspruch 1, wobei das Baffle Streben einschließt, die zu einem Korb konfiguriert sind, der ein hohles Innenvolumen aufweist, wobei das Baffle ferner einen Rückhalteabschnitt einschließt, der konfiguriert ist, um mit mindestens einen ersten Abschnitt eines funktionell defizienten nativen Segels in Eingriff zu stehen und diesen zurückzuhalten, während ein zweiter Abschnitt des funktionell defizienten nativen Segels beweglich bleibt, wobei sich das Baffle radial nach innen in das zentrale Lumen erstreckt, um sich einer geschlossenen Position für das funktionell defiziente Segel anzunähern.

3. Vorrichtung nach Anspruch 1, ferner umfassend eine Vielzahl von Reibungselementen (112), die an Abschnitten des Vorhoffixierungselements oder des Baffle bereitgestellt sind; oder
wobei das Baffle eine Vielzahl von anterioren Streben (116) und eine Vielzahl von posterioren Streben (118) umfasst, mit Spalten, die zwischen benachbarten der anterioren und posterioren Streben eingefügt sind.

4. Vorrichtung nach Anspruch 2, wobei die medialen und lateralen Seiten des Vorhoffixierungselements keine Reibungselemente einschließen.

5. Vorrichtung nach Anspruch 1, wobei das Baffle einen Korb umfasst, der einen hohlen Innenraum umschließt; oder wobei das Baffle einen biokompatiblen Schaum umfasst.

6. Vorrichtung nach Anspruch 2, umfassend:
eine Gewebeabdeckung (122), die das Baffle mindestens teilweise umgibt; und
der Korb eine Öffnung aufweist, die durch das Gewebe abgedeckt ist oder die nicht durch das Gewebe abgedeckt ist.

7. Vorrichtung nach Anspruch 1, wobei der anteriore Abschnitt des Baffle eine glatte Oberfläche und/oder eine konvexe Form aufweist; und/oder
wobei der posteriore Abschnitt des Baffle bemessen und konfiguriert ist, um mit einer zentralen Wellung eines nativen Mitralklappensegels, das drei Wellungen aufweist, in Eingriff zu stehen, während die verbleibenden zwei Wellungen beweglich bleiben.

8. Vorrichtung nach Anspruch 1, ferner umfassend:
einen ersten Satz von Fadenschlaufen (252), die umlaufend um das Baffle angeordnet sind, wobei jede Fadenschlaufe des ersten Satzes von Fadenschlaufen ein Lumen aufweist; und
einen ersten Faden (252S), der in dem Lumen der Fadenschlaufen des ersten Satzes von Fadenschlaufen angeordnet ist und benachbarte Fadenschlaufen des ersten Satzes von Fadenschlaufen miteinander verbindet;
wobei ein Durchmesser des Baffle durch Festziehen des ersten Fadens eingestellt wird; und/oder
ferner umfassend:
einen zweiten Satz von Fadenschlaufen (254), die umlaufend um das Vorhoffixierungselement angeordnet sind, wobei jede Fadenschlaufe des zweiten Satzes von Fadenschlaufen ein Lumen aufweist; und
einen zweiten Faden (254S), der in dem Lumen der Fadenschlaufen des zweiten Satzes von Fadenschlaufen angeordnet ist und benachbarte Fadenschlaufen des zweiten Satzes von Fadenschlaufen miteinander verbindet;
wobei ein Durchmesser des Vorhoffixierungselements durch Festziehen des zweiten Fadens eingestellt wird; und/oder
ferner umfassend:
eine oder mehrere dritte Fadenschlaufen (256), die an dem Baffle bereitgestellt sind,
einen dritten Faden (256S), der in dem Lumen der einen oder der mehreren dritten Fadenschlaufen angeordnet ist;
wobei das Ziehen an dem dritten Faden das Baffle verlagert.

9. Vorrichtung nach Anspruch 1, ferner umfassend mindestens eine Gewebeabdeckung (108), die an dem Vorhoffixierungselement befestigt ist und dieses mindestens teilweise überspannt, wobei die Gewebeabdeckung ein Einwachsen von Gewebe erleichtert.

10. Vorrichtung nach Anspruch 1, ferner umfassend mindestens ein Gewebesegment aus Gewebe, das von dem distalen Ende des Vorhoffixierungselements herabhängt; optional,
ferner umfassend ein Vorspannelement (206), das an dem mindestens einen Gewebesegment befestigt ist und das Gewebesegment von dem zentralen Lumen weg vorspannt.

11. Vorrichtung nach Anspruch 1, wobei das Vorhoffixierungselement mindestens zwei Reihen von Zellen umfasst; oder
wobei:
das Vorhoffixierungselement ferner eine Reihe von Chevrons umfasst;
mindestens zwei der Chevrons eine Durchgangsloch einschließen; und
einen Faden, der durch das Durchgangsloch gefädelt ist;
wobei das Festziehen des Fadens einen Durchmesser des Vorhoffixierungselements einstellt.

12. Vorrichtung nach Anspruch 1, wobei das Vorhoffixierungselement eine kegelstumpfförmige Form aufweist; optional,
wobei das Vorhoffixierungselement konfiguriert ist, um ausschließlich mit einer Vorhofwand der nativen Herzklappe in Eingriff zu stehen.

13. Vorrichtung nach Anspruch 12, wobei eine Größe des Vorhoffixierungselements, das durch äußere Kräfte uneingeschränkt ist, größer als eine Größe eines nativen Vorhofs in einer Diastole ist, optional,
wobei die Vorrichtung relativ zu der nativen Herzklappe ausschließlich durch das Vorhoffixierungselement und die Umlenkwand fixiert ist.

14. Vorrichtung nach Anspruch 1, wobei das Vorhoffixierungselement eine anteriore Seite und eine posteriore Seite aufweist, ein proximales Ende der anterioren Seite des Vorhoffixierungselements vertikal gegenüber einem proximalen Ende der posterioren Seite des Vorhoffixierungselements versetzt ist, und/oder
wobei das Vorhoffixierungselement eine asymmetrische Form aufweist, wobei eine Länge einer anterioren Seite länger als eine Länge einer posterioren Seite des Vorhoffixierungselements derart ist, dass die anteriore Seite des Vorhoffixierungselements in einer vertikalen Richtung höher als die posteriore Seite des Vorhoffixierungselements ist.

15. Vorrichtung nach Anspruch 1, wobei eine posteriore Seite des Vorhoffixierungselements steifer als eine anteriore Seite des Vorhoffixierungselements ist; optional,
wobei das Vorhoffixierungselement eine Vielzahl von miteinander verbundenen Streben einschließt, die mehrere Zellen ausbilden, wobei die Streben auf der posterioren Seite des Vorhoffixierungselements mindestens eines von dicker sind, breiter sind und/oder schmalere Spalte zwischen benachbarten Streben als die Streben auf der anterioren Seite des Vorhoffixierungselements aufweisen.

## Revendications

1. Dispositif de réparation de valve cardiaque (100) comprenant :
un élément de fixation auriculaire (102) ayant un maillage expansible conçu pour avoir une forme ovale ou circulaire dans une configuration déployée, l'élément de fixation auriculaire (102) définissant une lumière centrale (102L) et étant conçu pour venir en contact avec une paroi auriculaire en amont d'un anneau d'une valve cardiaque native ; et
un déflecteur (114) s'étendant depuis une partie de l'élément de fixation auriculaire (102), le déflecteur (114) ayant une partie antérieure avec une surface atraumatique conçue pour effectuer une coaptation avec au moins une partie d'un ou plusieurs feuillets natifs de la valve cardiaque native, une partie postérieure conçue pour venir en prise avec et déplacer au moins une partie d'un autre feuillet natif de la valve cardiaque native, dans lequel le déflecteur (114) s'étend radialement vers l'intérieur depuis l'élément de fixation auriculaire (102) dans la lumière centrale pour se rapprocher d'une position fermée du feuillet natif déplacé.

2. Dispositif selon la revendication 1, dans lequel le déflecteur comporte des entretoises conçues en un panier ayant un volume intérieur creux, le déflecteur comportant en outre une partie de retenue conçue pour venir en prise avec et retenir au moins une première partie d'un feuillet natif fonctionnellement déficient tout en laissant une seconde partie du feuillet natif fonctionnellement déficient mobile, le déflecteur s'étend radialement vers l'intérieur dans la lumière centrale pour se rapprocher d'une position fermée pour le feuillet fonctionnellement déficient.

3. Dispositif selon la revendication 1, comprenant en outre une pluralité d'éléments de frottement (112) prévus sur des parties de l'élément de fixation auriculaire ou du déflecteur ; ou
dans lequel le déflecteur comprend une pluralité d'entretoises antérieures (116) et une pluralité d'entretoises postérieures (118) avec des espaces interposés entre des entretoises adjacentes parmi les entretoises antérieures et postérieures.

4. Dispositif selon la revendication 2, dans lequel les côtés médial et latéral de l'élément de fixation auriculaire ne comportent aucun élément de frottement.

5. Dispositif selon la revendication 1, dans lequel le déflecteur comprend un panier renfermant un intérieur creux ; ou dans lequel le déflecteur comprend une mousse biocompatible.

6. Dispositif selon la revendication 2, comprenant :
un revêtement de tissu (122) entourant au moins partiellement le déflecteur ; et
le panier a une embouchure qui est recouverte par le tissu ou qui n'est pas recouverte par le tissu.

7. Dispositif selon la revendication 1, dans lequel la partie antérieure du déflecteur a une surface lisse et/ou une forme convexe ; et/ou
dans lequel la partie postérieure du déflecteur est dimensionnée et conçue pour venir en prise avec un feston central d'un feuillet de valve mitrale native ayant trois festons, tout en laissant les deux festons restants mobiles.

8. Dispositif selon la revendication 1, comprenant en outre :
un premier ensemble de boucles de suture (252) disposé de manière circonférentielle autour du déflecteur, chaque boucle de suture du premier ensemble de boucles de suture ayant une lumière ; et
une première suture (252S) disposée dans la lumière des boucles de suture du premier ensemble de boucles de suture et inter-reliant des boucles de suture adjacentes du premier ensemble de boucles de suture ;
dans lequel un diamètre du déflecteur est ajusté par serrage de la première suture ; et/ou
comprenant en outre :
un second ensemble de boucles de suture (254) disposé circonférentiellement autour de l'élément de fixation auriculaire, chaque boucle de suture du second ensemble de boucles de suture ayant une lumière ; et
une deuxième suture (254S) disposée dans la lumière des boucles de suture du second ensemble de boucles de suture et inter-reliant des boucles de suture adjacentes du second ensemble de boucles de suture ;
dans lequel un diamètre de l'élément de fixation auriculaire est ajusté par serrage de la deuxième suture ; et/ou
comprenant en outre :
une ou plusieurs troisièmes boucles de suture (256) prévues sur le déflecteur,
une troisième suture (256S) disposée dans la lumière de la ou des troisièmes boucles de suture ;
dans lequel la traction sur la troisième suture déplace le déflecteur.

9. Dispositif selon la revendication 1, comprenant en outre au moins un revêtement de tissu (108) fixé à et recouvrant au moins partiellement l'élément de fixation auriculaire, dans lequel le revêtement de tissu facilite la croissance tissulaire interne.

10. Dispositif selon la revendication 1, comprenant en outre au moins un segment de tissu s'étendant à partir de l'extrémité distale de l'élément de fixation auriculaire ; éventuellement,
comprenant en outre un élément de sollicitation (206) fixé à l'au moins un segment de tissu et sollicitant le segment de tissu à l'écart de la lumière centrale.

11. Dispositif selon la revendication 1, dans lequel l'élément de fixation auriculaire comprend au moins deux rangées de cellules ; ou
dans lequel :
l'élément de fixation auriculaire comprend en outre une rangée de chevrons ;
au moins deux des chevrons comportent un trou traversant ; et
une suture enfilée à travers le trou traversant ;
dans lequel le serrage de la suture ajuste un diamètre de l'élément de fixation auriculaire.

12. Dispositif selon la revendication 1, dans lequel l'élément de fixation auriculaire a une forme tronconique ; éventuellement,
dans lequel l'élément de fixation auriculaire est conçu pour venir en prise uniquement avec une paroi auriculaire de la valve cardiaque native.

13. Dispositif selon la revendication 12, dans lequel une taille de l'élément de fixation auriculaire non contraint par des forces externes est supérieure à une taille d'un atrium natif en diastole, éventuellement,
dans lequel le dispositif est fixé par rapport à la valve cardiaque native uniquement par l'élément de fixation auriculaire et le déflecteur.

14. Dispositif selon la revendication 1, dans lequel l'élément de fixation auriculaire a un côté antérieur et un côté postérieur, une extrémité proximale du côté antérieur de l'élément de fixation auriculaire est décalée verticalement par rapport à une extrémité proximale du côté postérieur de l'élément de fixation auriculaire, et/ou
dans lequel l'élément de fixation auriculaire a une forme asymétrique avec une longueur d'un côté antérieur étant supérieure à une longueur d'un côté postérieur de l'élément de fixation auriculaire de telle sorte que le côté antérieur de l'élément de fixation auriculaire est plus haut dans une direction verticale que le côté postérieur de l'élément de fixation auriculaire.

15. Dispositif selon la revendication 1, dans lequel un côté postérieur de l'élément de fixation auriculaire est plus rigide qu'un côté antérieur de l'élément de fixation auriculaire ; éventuellement,
dans lequel l'élément de fixation auriculaire comprend une pluralité d'entretoises inter-reliées formant plusieurs cellules, les entretoises sur le côté postérieur de l'élément de fixation auriculaire étant au moins plus épaisses, plus larges et/ou ayant des espaces plus étroits entre des entretoises adjacentes que les entretoises sur le côté antérieur de l'élément de fixation auriculaire.
